# EUROPEAN PATENT APPLICATION

(11) **EP 4 438 714 A1**
(43) Date of publication of application: **02.10.2024**
(21) Application number: 22897877.1
(22) Date of filing: 24.11.2022
(51) Int. Cl.: C12N 1/00, C12N 1/16, C12P 7/46

(54) **ACID-RESISTANT YEAST STRAIN FOR HIGH-YIELD PRODUCTION OF L-MALIC ACID, AND CONSTRUCTION METHOD THEREFOR AND USE THEREOF**

(30) Priority: 24.11.2021 CN 202111406618
(71) Applicant: Tianjin Institute of Industrial Biotechnology, Chinese Academy of Sciences, Tianjin Airport Economic Area Tianjin 300308 (CN)
(72) Inventor: ZHANG, Xueli, Tianjin 300308 (CN); FAN, Feiyu, Tianjin 300308 (CN); XI, Yongyan, Tianjin 300308 (CN); XU, Hongtao, Tianjin 300308 (CN)
(74) Representative: Nederlandsch Octrooibureau
(86) International application number: PCT/CN2022/133964
(87) International publication number: WO 2023/093794

(57) **Abstract**

The present invention provides a genetically modified malic acid producing yeast strain, wherein the strain has or has enhanced malate transport protein activity and has or has enhanced NADPH-dependent malate dehydrogenase (EC 1.1.1.82) activity, optionally also has or has enhanced at least one of the following activities: (i) pyruvate carboxylase (EC 6.4.1.1) activity, (ii) phosphoenolpyruvate carboxykinase (EC 4.1.1.49) activity, (iii) phosphoenolpyruvate carboxylase activity, and (iv) biotin transport protein activity; and a preparation method thereof, a method for producing L-malic acid using the same, and use thereof.

## Description

### Technical Field

The present invention relates to the field of biotechnology, and particularly to a novel acid-resistant yeast strain for efficient producuction of L-malic acid, and a construction method therefor and use thereof.

### Background Art

Malic acid, also known as 2-hydroxybutanedioic acid, is an intermediate metabolite of the tricarboxylic acid cycle in organisms. Malic acid has two stereisomers (L-type and D-type) due to the presentce of an asymmetric carbon atom in the molecule. However, naturally found malic acid is L-malic acid. As an important C4 bulk chemical, L-malic acid has a wide variety of uses.

At present, the methods of producing malic acid mainly include chemical method, enzyme catalysis method and biological fermentation method. In the chemical synthesis method, malic acid is synthesized mainly using cis-butenedioic acid or fumaric acid from fossil fuels, under the conditions of high temperature and high pressure. The chemical method has the advantages of a mature process and low cost, but has the problem of poor catalytic stereoselectivity, and the resulting malic acid product is mixture of L-type and D-type, which makes it difficult to obtain L-malic acid with high-purity. In the enzyme catalysis method, L-malic acid is formed mainly by catalyzing the hydration of fumaric acid with the aid of fumarase. This method is clean and efficient, but the high costs for preparing enzyme and substrate is disadvantageous to large-scale production. At present, the strains mainly used in the microbial fermentation method are filamentous fungi, including *Aspergillus oryzae, Aspergillus flavus, Ustilago trichophora,* etc. Although filamentous fungal fermentation can achieve a higher titer of L-malic acid, there are also some problems, for example slow growth of filamentous fungi, complex and time-consuming of genetic modification, poor cell morphology homogeneity during liquid deep fermentation, low mechanical shear force tolerance, and easy mycelial agglomeration, which render the fermentation process difficult to control. In addition, some fungal strains themselves further have mycotoxins (such as aflatoxin), which pose a potential safety risk. Compared with filamentous fungi, yeast grows faster and is easy to be genetically modified, making it an ideal production strain. At present, some yeast strains, for example *Saccharomyces cerevisiae, Zygosaccharomyces rouxii, Aureobasidium pullulans* can already achieve higher titer of L-malic acid production, but the yield of sugar-acid is generally low, and the production cost is still high.

In addition, the cost of organic acid isolation and purification accounts for about 50% of that of the entire fermentation process. The traditional L-malic acid production process requires calcium carbonate as a neutralizing agent to precipitate L-malic acid in a form of calcium malate out of the fermentation system. Calcium malate needs to be further converted to L-malic acid by replacement reaction through sulfuric acid, and the entire flow process can generate a large amount of calcium sulfate solid wastes, which is not friendly to ecological environment. Some acid-resistant yeast cells can normally grow in harsh environments with a pH value of less than 3.0. Under such conditions, malic acids are mostly present in the form of undissolved moleculars (malic acid pKa1=3.46), which can greatly simplify the flow of downstream isolation and purification and reduce the cost.

### Summary of the Invention

In the present invention, the high-efficiency production of L-malate is achieved by metabolic engineering modification by starting from an acid-resistant yeast *Pichia kudriαvzevii* CY902 strain (deposited in China General Microbiological Culture Collection Center (CGMCC) under the deposit number of CGMCC No. 20885) isolated from the epidermis of a wild fruit in Yunnan. Specifically, the modified strain can achieve the high-efficiency production of L-malate in a fermentation manner with no or less addition of a calcium carbonate neutralizing agent at pH < 3.5.

In one aspect, the present invention provides a genetically modified malate-producing yeast strain, having activity or enhanced activity of malate transport protein and having activity or enhanced activity of NADPH-dependent malate dehydrogenase, optionally further having activity or enhanced activity of at least one of the following: (i) pyruvate carboxylase activity, (ii) phosphoenolpyruvate carboxykinase activity, (iii) phosphoenolpyruvate carboxylase activity, and (iv) biotin transport protein activity.

Preferably, said NADPH-dependent malate dehydrogenase is derived from a plant, preferably a C4 plant, more preferably a plant of *Gramineae, Cyperaceae, Compositae, Euphorbiaceae, Chenopodiaceae, Portulacaceae* or *Amaranthaceae,* or is derived from the genus *Euglenα* or *Thermobacillus,* more preferably from *Sorghum bicolor, Zea mays, Saccharum officinαrum, Pisum sativum, Cicer arietinum, Spinαciα oleracea, Euglenα gracilis* or *Methanothermobacter thermautotrophicu.*

In one embodiment, said genetically modified malate-producing yeast strain further has reduced activity of or inactivated pyruvate decarboxylase and/or NAD-dependent glycerol-3-phosphate dehydrogenase.

In one aspect, the present invention provides a method for preparing a genetically modified malate-producing yeast strain, comprising conferring malate transport protein activity on the strain or enhancing malate transport protein activity of the strain, and conferring NADPH-dependent malate dehydrogenase activity on the strain or enhancing NADPH-dependent malate dehydrogenase of the strain, optionally further comprising conferring or enhancing at least one of the following activities: (i) pyruvate carboxylase activity, (ii) phosphoenolpyruvate carboxykinase activity, (iii) phosphoenolpyruvate carboxylase activity, and (iv) biotin transport protein activity. Preferably, said NADPH-dependent malate dehydrogenase is derived from a plant, preferably a C4 plant, more preferably a plant of the family Gramineae, Cyperaceae, Compositae, Euphorbiaceae, Chenopodiaceae, Portulacaceae and Amaranthaceae, or is derived from the genus *Euglenα* or *Thermobacillus,* more preferably from *sorghum bicolor, Zea mays, Saccharum officinarum, Pisum sativum, Cicer αrietinum, Spinαciα oleracea, Euglenα gracilis* or *Methanothermobacter thermautotrophicus.*

In one embodiment, said method further comprises attenuating or inactivating pyruvate decarboxylase and/or the NAD-dependent glycerol-3-phosphate dehydrogenase in the strain.

In one aspect, the present invention provides a method for producing L-malate, comprising (preferably at pH <3.5 for example at a pH value in the range of 2.0-3.5 and/or with no or less addition of a neutralizing agent) culturing the genetically modified malate-producing yeast strain of the present invention and/or a genetically modified malate-producing yeast strain obtained by the method for preparing a genetically modified malate-producing yeast strain of the present invention.

In one aspect, the present invention provides use of the genetically modified malate-producing yeast strain of the present invention and/or a genetically modified malate-producing yeast strain obtained by the method for preparing a genetically modified malate-producing yeast strain of the present invention in the production of L-malate, preferably in the production of L-malate at pH <3.5 for example at a pH value in the range of 2.0-3.5 and/or with no or less addition of a neutralizing agent.

### Detailed Description of the Invention

Unless otherwise defined, the technical and scientific terms used herein have the meanings commonly understood by those skilled in the art, for example by referring to Singleton et al., DICTIONARY OF MICROBIOLOGY AND MOLECULAR BIOLOGY 2nd ed., J. Wiley & Sons (New York, NY 1994); Sambrook et al., MOLECULAR CLONING, A LABORATORY MANUAL, Cold Springs Harbor Press (Cold Springs Harbor, NY 1989).

As used herein, the term "genetically modified" means that a strain artificially modified by biological means has one or more modifications, for example gene deletion, amplification or mutation, compared with its initial strain prior to modification, thereby possessing modified biological properties such as improved production performance. As used herein, the term "initial strain" can be a natural strain to be genetically modified or a strain having other genetic modifications.

As used herein, the term "malate-producing yeast strain" refers to a yeast that can produce malate(s) (for example L-malate) under suitable conditions (for example via fermentation) and secrete the malate(s) into an extracellular medium, preferably, the amount of L-malate in said malates is above 50% of that of the malates, for example at least 60%, at least 70%, at least 80%, at least 90%, at least 91%, at least 92%, at least 93%, at least 94%, at least 95%, at least 96%, at least 97%, at least 98%, at least 99% or more, or even 100%. The malate-producing yeast strain has a protein that can transport malate to the outside of the cell, and therefore malate can be secreted to the outside of the cell after being produced. Suitable malate transport proteins used for the given yeast strains are known in the art, for example including, but is not limited to, the malate transport protein SpMAE1 of *Schizosaccharomyces pombe* and the C4-dicarboxylate transport protein C4T318 of *Aspergillus oryzae.*

Malate-producing yeasts are known in the art, including for example, but is not limited to, *Zygosaccharomyce, Torulopsis, Candida, Pichia, Rhodotroula, Saccharomyces, Yarrowia,* etc. In one embodiment, said malate-producing yeast strain is a strain from *Pichia.* In one preferred embodiment, said malate-producing yeast strain is *Pichia kudriαvzevii,* for example the *Pichia kudriαvzevii* deposited in China General Microbiological Culture Collection Center (CGMCC) under the deposit number of CGMCC No. 20885 on October 14, 2020.

As used herein, "having activity/activites" refers to having detectable activity/activites compared with a reference (for example an initial strain or a wild-type strain) without such activity/activites.

As used herein, "having enhanced activity/activites" means that the activity (activites) is increased by at least 5%, at least 10%, at least 20%, at least 30%, at least 40%, at least 50%, at least 60%, at least 70%, at least 80%, at least 90%, at least 100%, at least 150%, at least 200%, at least 250%, at least 300% or more, compared with a reference (for example an initial strain or a wild-type strain) with this activity.

The activity of a protein (for example an enzyme) may be generated or enhanced through any suitable methods known in the art, for example including but not limited to, expressing or over-expressing (for example via a vector such as a plasmid) a corresponding gene encoding the protein in the strain, introducing mutation that leads to an increase in the activity of the protein, etc.

In some embodiments, in the genetically modified malate-producing yeast strain of the present invention, one or more copies of a target gene or a homologous gene thereof can be integrated into the genome (for example through homologous recombination), optionally at any locus of the genome (as long as such the integration does not significantly negatively affect the growth and production of the strain), for example where one copy of any gene in the genome is replaced by one or more copies of the target gene or the homologous gene thereof. Those skilled in the art know how to integrate transgenes and select strains in which transgenes are integrated.

As used herein, the term "activity-reduced or inactivated ..." or "reduced activity of or inactivated ..." means that the activity is reducted by at least 5%, at least 10%, at least 20%, at least 30%, at least 40%, at least 50%, at least 60%, at least 70%, at least 80%, at least 90%, at least 95%, at least 99% or more or even 100%, compared with reference activity (for example corresponding activity in an initial strain or a wild-type strain).

The activity of a protein (for example an enzyme) can be reduced or inactivated through any suitable means known in the art, for example including, but not limited to, using the attenuated or inactivated corresponding gene encoding the protein, introducing a mutation that leads to a reducted activity or inactivation of the protein, using the antagonist or inhibitor (for example an antibody, a ligand, etc.) of the protein.

As used herein, the term "attenuated or inactivated gene" means that gene activity, for example the expression level (when being used as a protein encoding gene) or the regulatory performance (when being used as a regulatory element), is reduced by at least 5% , at least 10%, at least 20%, at least 30%, at least 40%, at least 50%, at least 60%, at least 70%, at least 80%, at least 90%, at least 95%, at least 99% or more compared with reference (for example a corresponding gene in an initial strain or a wild-type strain), or even is undetectable. In the case of a gene encoding a protein such as an enzyme, "attenuated or inactivated gene" also encompasses that the activity level of the protein expressed by this gene is reduced, for example reduced by at least 5% at least 10%, at least 20%, at least 30%, at least 40%, at least 50%, at least 60%, at least 70%, at least 80%, at least 90%, at least 95%, at least 99% or more, or even 100%, compared with the activity level of a corresponding protein in an initial strain or a wild-type strain.

Herein, the reference can be a wild-type microorganism or a microorganism prior to performing the desired genetic manipulation (for example an initial microorganism to be used for genetic manipulation to increase gene activity). Herein, parental microorganisms and initial microorganisms can be interchangeably used, and refer to the microorganisms to which the desired genetic manipulation (for example enhancing or attenuating gene or protein activity) is to be performed.

As used herein, malate dehydrogenase (EC 1.1.1.82 (NADPH-dependent)) is encoded by an *MDH* gene, and is invovled in conversion between oxaloacetate and malate. The NADPH-dependent malate dehydrogenase contributes to the conversion from oxaloacetate into malate, and in this reaction one molecule of NADPH is consumed. The generally used NADPH-dependent malate dehydrogenase sources include C4 plants (for example Gramineae, Cyperaceae, Compositae, Euphorbiaceae, Chenopodiaceae, Portulacaceae and Amaranthaceae plants), *Euglenα* and *Thermobαcillus,* etc, for example *Sorghum bicolor, Zea mays, Saccharum officinarum, Pisum sativum, Cicer αrietinum, Spinαciα oleracea, Euglenα gracilis* or *Methanothermobacter thermautotrophicus,* etc. As used herein, "having activity or enhanced activity of NDAPH-dependent malate dehydrogenase" means that a strain has NDAPH-dependent malate dehydrogenase activity or increased NDAPH-dependent malate dehydrogenase activity that catalyzes the conversion of oxaloacetate into malate.

As used herein, the term "malate transport protein" refers to a protein capable of transporting intracellular malate to the outside of the cell, including for example, but not limited to, SpMAE1 protein of *Schizosaccharomyces pombe* (Uniprot database retrieval number: P50537) and C4-dicarboxylate transport protein C4T318 of *Aspergillus oryzae* (Gene ID: 5992883) and *AsDct* of *Actinobacillus succinogenes* (NCBI Reference Sequence: WP_012073722.1), etc. Herein, "having activity or enhanced activity of malate transport protein" means that a strain has activity or increased activity of transporting malate to the outside of the cell.

As used herein, the dicarboxylate transport protein SpMAE1 protein of *Schizosaccharomyces pombe* is encoded by an *SpMAEl* gene, and the SpMAE1 protein contributes to transporting the intracellular dicarboxylate to the outside of the cell. Herein, the term "having activity or enhanced activity of SpMAE1" means that a strain has activity or increased activity of transporting dicarboxylate to the outside of the cell.

As used herein, pyruvate carboxylase (EC 6.4.1.1) is encoded by a *PYC* gene. The pyruvate carboxylase is invoved in the interconversion between oxaloacetate and pyruvate in the process of gluconeogenesis, and contributes to the conversion of pyruvate and carbon dioxide into oxaloacetate. The generally used pyruvate carboxylase sources include fungi, especially yeasts and filamentous fungi, preferably *Saccharomyces cerevisiae, Pichia kudriαvzevii, Aspergillus oryzae, kluyveromyces marxianus,* etc. It is known that one *PYC* gene, i.e., *PYC1* gene, is present in *Pichia kudriavzevii.* Herein, the term "having activity or enhanced activity of pyruvate carboxylase" means that a strain has activity or increased activity of converting pyruvate into oxaloacetate.

As used herein, phosphoenolpyruvate carboxykinase (EC4.1.1.49) is encoded by a *PCK* gene. The phosphoenolpyruvate carboxykinase is involved in interconversion between oxaloacetate and phosphoenolpyruvate, and contributes to the conversion of phosphoenolpyruvate and carbon dioxide into oxaloacetate. The generally used phosphoenolpyruvate carboxykinase sources include *Saccharomyces cerevisiae* and *Escherichia coli.* It is known that one *PCK* gene, i.e., *PCK1* gene, is present in *Pichia kudriavzevii.* Herein, the term "having activity or enhanced activity of phosphoenolpyruvate carboxykinase" means that a strain has activity or increased activity of converting phosphoenolpyruvate into oxaloacetate.

The phosphoenolpyruvate carboxylase (PPC, EC 4.1.1.31) contributes to the conversion of phosphoenolpyruvate and carbon dioxide into oxaloacetate. The plant sources (for example Bacillariophyta and Angiospermophyta plants) and bacterial sources (for example Proteobacteria), preferably *Escherichia coli, Phaeodactylum tricornutum, Sorghum bicolor, Zea mays,* rice sources, etc., are generally used. Herein, the term "having activity or enhanced activity of phosphoenolpyruvate carboxylase" means that a strain has activity or increased activity of converting phosphoenolpyruvate into oxaloacetate.

As used herein, the term "biotin (vitamin H) transport protein" refers to a protein capable of conveting the biotin from the outside of the cell into the cell. The suitable biotin transport protein for a given yeast strain is known in the art. The biotin transport protein is generally derived from fungi and bacteria, especially yeasts, *Pseudomonas* and *Rhizobium,* further preferably from *Saccharomyces cerevisiae, Schizosaccharomyces pombe, Candida glabrata, Kluyveromyces marxianus, Pseudomonas mutabilis, Sinorhizobium meliloti,* etc. Herein, the term "having activity or enhanced activity of biotin transport protein" means that a strain has activity or increased activity of transporting biotin from a culture medium into the cell.

In one embodiment, the biotin transport protein is biotin transport protein SpVHT1 of *Schizosaccharomyces pombe* (Uniprot database search number: 013880), which is encoded by *SpVHT1* gene and contributes to transporting the biotin from the culture medium into the cell. Herein, the term "having activity or enhanced activity of biotin transport protein SpVHT1" means that a strain has activity or increased activity of transporting the biotin from the culture medium into the cell.

As used herein, pyruvate decarboxylase (EC 4.1.1.43) is encoded by a *PDC* gene, and is involved in the decarboxylation process of pyruvate in the pathway of ethanol synthesis. It is known that one PDC gene is present in *Pichia kudriαvzevii* is *PDC1* gene. Herein, the term "activity-reduced or inactivated pyruvate decarboxylase" refers to a reduction or loss in pyruvate decarboxylation activity of this enzyme.

As used herein, NAD-dependent glycerol-3-phosphate dehydrogenase (EC 1.1.1.8) is encoded by a *GPD* gene, and is involved in interconversion between glycerone phosphate and glycerol 3-phosphate in the pathway of glycerol synthesis. It is known that one *GPD* gene, i.e., *GPD1* gene, is present in *Pichia kudriavzevii.* Herein, the "activity-reduced or inactivated NAD-dependent glycerol-3-phosphate dehydrogenase" refers to a reduction or loss in the activity of this enzyme that catalyzes interconversion between glycerone phosphate and glycerol 3-phosphate.

As used herein, orotidine 5'-phosphate decarboxylase (EC 4.1.1.23) is encoded by a *URA3* gene, and is involved in decarboxylation reaction of orotidine 5'-phosphate in the process of pyrimidine synthesis. It is known that one *URA3* gene is present in *Pichia kudriavzevii.* Herein, the term "activity-reduced or inactivated orotidine 5'-phosphate decarboxylase" refers to a reduction or loss in the activity of this enzyme that catalyzes orotidine 5'-phosphate decarboxylation reaction.

As used herein, monocarboxylate permease (NCBI Reference Sequence: XP_029320775.1) is encoded by an *MCH4* gene. Herein, the term "activity-reduced or inactivated monocarboxylate permease" refers to a reduction or loss in catalytic activity of this enzyme. In one embodiment, the *MCH4* gene in the strain is knocked out, for example through homologous recombination.

As used herein, a *JEN2* gene encodes dicarboxylate transport protein, which is involved in a process of transporting dicarboxylate from the culture medium into the cell. It is known that there are two *JEN2* genes (*JEN*2-1 (encoding a polypeptide of SEQ ID NO: 14) and *JEN*2-2 (encoding a polypeptideof SEQ ID NO: 15)) in *Pichia kudriavzevii.* Herein, the term "activity-reduced or inactivated dicarboxylate transport protein" refers to a reduction or loss in the cell activity of transporting dicarboxylate from the culture medium into the cell.

As used herein, the term "neutralizing agent" refers to a reagent that precipitates malate in a form of calcium malate out of a fermentation system. It is known in the art that substances that can be used as neutralizing agents include for example but are not limited to calcium carbonate.

As used herein, the term "no or less addition of a neutralizing agent" refers to an amount of a neutralizing agent that is at least 5%, at least 10%, at least 20%, at least 30%, at least 40%, at least 50%, at least 60%, at least 70%, at least 80%, at least 90%, at least 95%, at least 99% or more, or even 100% lower than the amount of the neutralizing agent known in the art that is added for fermentation production of malate. For example, the amount of a neutralizing agent added in the method of the present invention can be 0-60 g/L.

As used herein, the terms "polypeptide", "amino acid sequence", "peptide" and "protein" can be interchangeably used herein, and refer to an amino acid chain with any length, which may comprise a modified amino acid and/or may be interrupted by a non-amino acid. These terms also encompass an amino acid chain modified by natural or artificial intervention; for example, disulfide bond formation, glycosylation, lipidation, acetylation, phosphorylation or any other manipulations or modifications, such as conjugation with a marker component.

As used herein, the expressions "gene", "nucleic acid sequence", "polynucleotide" and "nucleotide sequence" can be interchangeably used, and refer to a nucleotide chain comprising DNA and/or RNA. The term "expression of a gene" or "gene expression" means that a DNA region operatively linked to a suitable regulatory region, especially a promoter, is transcripted into RNA having biological activity, and RNA can be translated into a protein or peptide having biological activity.

As used herein, the term "degenerate sequence" refers to a nucleotide sequence that encodes the same amino acid sequence as that encoded by a specified sequence but has a different nucleotide sequence, due to degeneracy of genetic codons.

As used herein, the terms "homology", "sequence identity" and the like can be interchangeably used herein. The sequence identity can be detected by alignment of the numbers of the identical nucleotide bases between a polynucleotide and a reference polynucleotide, for example determined by a standard arrangement alignment algorithm program using default gap penalties established by each supplier. Whether or not two nucleic acid molecules have at least 80%, at least 85%, at least 90%, at least 95%, at least 96%, at least 97%, at least 98%, or at least 99% "identical" nucleotide sequence can be determined by using a known computer algorithm, such as BLASTN, FASTA, DNAStar and Gap (University of Wisconsin Genetics Computer Group (UWG), Madison WI, USA). For example, the identity percentage of a nucleic acid molecule can be determined, for example, by aligning sequence information using GAP computer program (e.g., Needleman et al. J. Mol. Biol. 48: 443 (1970), revised by Smith and Waterman (Adv. Appl. Math. 2: 482 (1981)). In brief, the GAP program defines similarity depending on the number of aligned similar symbols (i.e., nucleotides) divided by the total number of symbols in the shorter sequence of the two sequences.

As used herein, malic enzyme (EC 1.1.1.38) is encoded by *MAE1* gene, and contributes to conversion of malate into pyruvate via oxidatation and decarboxylation. Herein, the term "activity-reduced or inactivated malic enzyme" refers to a reduction or loss in the activity of this enzyme for converting malate into pyruvate via oxidation and decarboxylation.

As used herein, the *Pk2365* gene encodes a bifunctional enzyme of oxaloacetate decarboxylase and 3-hydroxy-3-methylglutarate aldolase (EC 4.1.3.17 or 4.1.1.112). Herein, the term "activity-reduced or inactivated bifunctional enzyme of oxaloacetate decarboxylase and 3-hydroxy-3-methylglutarate aldolase" refers to a reduction or loss in the activity of this enzyme for catalyzing oxaloacetate decarboxylation and aldol condensation of 3-hydroxy-3-methylglutarate.

In one aspect, the present invention provides a genetically modified malate-producing yeast strain, having activity or enhanced activity of malate transport protein, for example, SpMAE1 protein activity, and having activity or enhanced activity of NADPH-dependent malate dehydrogenase, optionally, further having activity or enhanced activity of at least one of the following: (i) pyruvate carboxylase (EC 6.4.1.1), (ii) phosphoenolpyruvate carboxykinase (EC 4.1.1.49), (iii) phosphoenolpyruvate carboxylase (EC 4.1.1.31), preferably *Escherichia coli* phosphoenolpyruvate carboxylase, and (iv) biotin transport protein. The "at least one" includes any one or two or three or all four activities selected therefrom.

In one embodiment, the present invention provides a genetically modified malate-producing yeast strain, having activities or enhanced activities of malate transport protein such as SpMAE1 protein, NADPH-dependent malate dehydrogenase, and pyruvate carboxylase.

In one embodiment, the present invention provides a genetically modified malate-producing yeast strain, having activities or enhanced activities of malate transport protein such as SpMAE1 protein, NADPH-dependent malate dehydrogenase, and phosphoenolpyruvate carboxykinase.

In one embodiment, the present invention provides a genetically modified malate-producing yeast strain, having activities or enhanced activities of malate transport protein such as SpMAE1 protein, NADPH-dependent malate dehydrogenase, and phosphoenolpyruvate carboxylase preferably *Escherichia coli* phosphoenolpyruvate carboxylase.

In one embodiment, the present invention provides a genetically modified malate-producing yeast strain, having activities or enhanced activities of malate transport protein such as SpMAE1 protein, NADPH-dependent malate dehydrogenase, and biotin transport protein.

In one embodiment, the present invention provides a genetically modified malate-producing yeast strain, having activities or enhanced activities of malate transport protein such as SpMAE1 protein, NADPH-dependent malate dehydrogenase, pyruvate carboxylase, and phosphoenolpyruvate carboxykinase.

In one embodiment, the present invention provides a genetically modified malate-producing yeast strain, having activities or enhanced activities of malate transport protein such as SpMAE1 protein, NADPH-dependent malate dehydrogenase, pyruvate carboxylase and phosphoenolpyruvate carboxylase preferably *Escherichia coli* phosphoenolpyruvate carboxylase.

In one embodiment, the present invention provides a genetically modified malate-producing yeast strain, having activities or enhanced activities of malate transport protein such as SpMAE1 protein, NADPH-dependent malate dehydrogenase, pyruvate carboxylase and biotin transport protein.

In one embodiment, the present invention provides a genetically modified malate-producing yeast strain, having activities or enhanced activities of malate transport protein such as SpMAE1 protein, NADPH-dependent malate dehydrogenase, phosphoenolpyruvate carboxykinase, and phosphoenolpyruvate carboxylase preferably *Escherichia coli* phosphoenolpyruvate carboxylase.

In one embodiment, the present invention provides a genetically modified malate-producing yeast strain, having activities or enhanced activities of malate transport protein such as SpMAE1 protein, NADPH-dependent malate dehydrogenase, phosphoenolpyruvate carboxykinase and biotin transport protein.

In one embodiment, the present invention provides a genetically modified malate-producing yeast strain, having activities or enhanced activities of malate transport protein such as SpMAE1 protein, NADPH-dependent malate dehydrogenase, phosphoenolpyruvate carboxylase preferably *Escherichia coli* phosphoenolpyruvate carboxylase, and biotin transport protein.

In one embodiment, the present invention provides a genetically modified malate-producing yeast strain, having activities or enhanced activities of malate transport protein such as SpMAE1 protein, NADPH-dependent malate dehydrogenase, pyruvate carboxylase, phosphoenolpyruvate carboxykinase and phosphoenolpyruvate carboxylase preferably *Escherichia coli* phosphoenolpyruvate carboxylase.

In one embodiment, the present invention provides a genetically modified malate-producing yeast strain, having activities or enhanced activities of malate transport protein such as SpMAE1 protein, NADPH-dependent malate dehydrogenase, pyruvate carboxylase, phosphoenolpyruvate carboxykinase and biotin transport protein.

In one embodiment, the present invention provides a genetically modified malate-producing yeast strain, having activities or enhanced activities of malate transport protein such as SpMAE1 protein, NADPH-dependent malate dehydrogenase, pyruvate carboxylase, phosphoenolpyruvate carboxylase preferably *Escherichia coli* phosphoenolpyruvate carboxylase, and biotin transport protein.

In one embodiment, the present invention provides a genetically modified malate-producing yeast strain, having activities or enhanced activities of malate transport protein such as SpMAE1 protein, NADPH-dependent malate dehydrogenase, phosphoenolpyruvate carboxykinase, phosphoenolpyruvate carboxylase preferably *Escherichia coli* phosphoenolpyruvate carboxylase, and biotin transport protein.

In one embodiment, the present invention provides a genetically modified malate-producing yeast strain, having activities or enhanced activities of malate transport protein such as SpMAE1 protein, NADPH-dependent malate dehydrogenase, pyruvate carboxylase, phosphoenolpyruvate carboxykinase, phosphoenolpyruvate carboxylase preferably *Escherichia coli* phosphoenolpyruvate carboxylase, and biotin transport protein.

In one embodiment, having activity (activites) or enahnced activity (activites) is achieved by expressing or over-expressing a corresponding encoding gene in the strain. Therefore, in one embodiment, a gene encoding malate transport protein, such as *SpMAE1* gene and/or NADPH-dependent malate dehydrogenase gene, is expressed or over-expressed in the genetically modified malate-producing yeast strain.

In one embodiment, the NADPH-dependent malate dehydrogenase is derived from a plant (preferably a C4 plant, more preferably Gramineae, Cyperaceae, Compositae, Euphorbiaceae, Chenopodiaceae, Portulacaceae and Amaranthaceae plants), *Euglenα* or *Thermobacillus,* preferably is derived from *Sorghum bicolor, Zea mays, Saccharum officinαrum, Pisum sativum, Cicer αrietinum, Spinαciα oleracea, Euglenα gracilis* or *Methanothermobacter thermautotrophicus,* more preferablyis derived from *Sorghum bicolor.*

In one embodiment, the NADPH-dependent malate dehydrogenase activity is generated or increased by expressing or over-expressing an *MDH* gene encoding NADPH-dependent malate dehydrogenase in the genetically modified malate-producing yeast strain. Preferably, the *MDH* gene encoding the NADPH-dependent malate dehydrogenase is preferably derived from a plant, preferably a C4 plant, more preferably a plant from the family Gramineae, Cyperaceae, Compositae, Euphorbiaceae, Chenopodiaceae, Portulacaceae or Amaranthaceae), or is derived from *Euglenα* or *Thermobacillus,* more preferably derived from *Sorghum bicolor, Zea mays, Saccharum officinαrum, Pisum sativum, Cicer αrietinum, Spinαciα oleracea, Euglenα gracilis* or *Methanothermobacter thermautotrophicus,* more preferably from *Sorghum bicolor.*

In one embodiment, said *MDH* gene encoding NADPH-dependent malate dehydrogenase comprises the sequence of SEQ ID NO: 1 and a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having NADPH-dependent malate dehydrogenase activity.

In one embodiment, said *MDH* gene encoding NADPH-dependent malate dehydrogenase is incorporated into the genome of the genetically modified malate-producing yeast (for example *Pichia kudriαvzevii),* for example at a position corresponding to *Pk2365* loci. Said *MDH* gene encoding NADPH-dependent malate dehydrogenase can be placed under the control of a suitable promoter (for example a promoter (for example as set forth in SEQ ID NO: 17) of *FBA1* gene) and/or terminator (for example a terminator (for example as set forth in SEQ ID NO: 18) of *INO1* gene).

In one embodiment, said malate transport protein is selected from the group consisting of SpMAE1 protein, C4T318 protein and AsDct protein. In one embodiment, the malate transport protein activity is generated or increased by expressing or over-expressing a gene encoding malate transport protein for example SpMAE1 protein, in the genetically modified malate-producing yeast strain. In one embodiment, said malate transport protein activity is generated or increased by expressing or over-expressing *SpMAE1* gene.

In one embodiment, said *SpMAE1* gene comprises the sequence of SEQ ID NO: 2 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence (for example from *Schizosaccharomyces pombe*) having malate transport protein activity. Optionally, said *SpMAE1* gene is incorporated into the genome of the genetically modified malate-producing yeast strain (for example *Pichia kudriavzevii*)*,* for example at a position of *MCH4* loci. The *SpMAE1* gene can be placed under the control of a suitable promoter (for example a promoter (for example as set forth in SEQ ID NO: 19) of *TDH3* gene) and/or terminator (for example a terminator (for example as set forth in SEQ ID NO: 20) of *GAL2* gene).

In a further embodiment, said genetically modified malate-producing yeast strain further has activity or enhanced activity of biotin transport protein such as SpVHT1. The biotin transport protein is generally derived from fungi and bacteria, especially yeasts, *Pseudomonas* and *Rrhizobium,* further preferably from *Saccharomyces cerevisiae, Schizosaccharomyces pombe, Candida glabrata, Kluyveromyces marxianus, Pseudomonas mutabilis* and *Sinorhizobium meliloti.*

The biotin transport protein activity can be generated or increased by expressing or over-expressing a gene encoding biotin transport protein such as SpVHT1 protein, in said genetically modified malate-producing yeast strain. Therefore, in one embodiment, said biotin transport protein activity can be generated or enhanced by expressing or over-expressing a gene encoding biotin transport protein, such as *SpVHT1* gene.

In one embodiment, said gene encoding biotin transport protein comprises the sequence of SEQ ID NO: 3 or a degenerate sequence thereof, or a nucleotide sequence having at least 75%, 80%, 85%, 90%, 91%, 92%, 93%, 94%, 95%, 96%, 97%, 98%, 99% or more identity thereto and encoding a polypeptide (for example derived from *Schizosaccharomyces pombe*) having said biotin transport protein activity. Optionally, the gene encoding biotin transport protein is incorporated into the genome of the genetically modified malate-producing yeast (for example *Pichia kudriαvzevii),* for example at a position of *PkMAE1* loci. The *SpVHT1* gene can be placed under the control of a suitable promoter (for example a promoter (for example as set forth in SEQ ID NO: 19) of *TDH3* gene) and/or terminator (for example a terminator (for example as set forth in SEQ ID NO: 20) of *GAL2* gene).

In a further embodiment, said genetically modified malate-producing yeast strain further has pyruvate carboxylase activity or enhanced pyruvate carboxylase activity. Said pyruvate carboxylase activity can be generated or enhanced by expressing or over-expressing a gene encoding pyruvate carboxylase. The pyruvate carboxylase can be derived from fungi, especially yeasts and filamentous fungi, preferably *Saccharomyces cerevisiae, Pichia kudriαvzevii, Aspergillus oryzae, Kluyveromyces marxianus,* etc. In one embodiment, the gene encoding pyruvate carboxylase can be selected from the group consisting of the *PYC* gene of *Aspergillus oryzae* and the *PYC1* gene of *Pichiα kudriavzevii.*

In one embodiment, said pyruvate carboxylase comprises the amino acid sequence of SEQ ID NO: 5 or an amino acid sequence having at least 75%, 80%, 85%, 90%, 91%, 92%, 93%, 94%, 95%, 96%, 97%, 98%, 99% or more identity thereto and having the pyruvate carboxylase activity.

In one embodiment, said pyruvate carboxylase comprises the amino acid sequence encoded by the sequence of SEQ ID NO: 4, or an amino sequence having at least 75%, 80%, 85%, 90%, 91%, 92%, 93%, 94%, 95%, 96%, 97%, 98%, 99% or more identity thereto and having pyruvate carboxylase activity.

In one embodiment, the gene encoding said pyruvate carboxylase encodes the amino acid sequence of SEQ ID NO: 5 or an amino acid sequence having at least 75%, 80%, 85%, 90%, 91%, 92%, 93%, 94%, 95%, 96%, 97%, 98%, 99% or more identity thereto and having said pyruvate carboxylase activity.

In one embodiment, the gene encoding said pyruvate carboxylase comprises the sequence of SEQ ID NO: 4 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having pyruvate carboxylase activity.

Optionally, the gene encoding pyruvate carboxylase is incorporated into the genome of the genetically modified malate-producing yeast (for example *Pichia kudriαvzevii),* for example at a position of *JEN2-1* loci. The gene encoding said pyruvate carboxylase can be placed under the control of a suitable promoter (for example a promoter (for example as set forth in SEQ ID NO: 19) of *TDH3* gene) and/or terminator (for example a terminator (for example as set forth in SEQ ID NO: 20) of *GAL2* gene).

In a further embodiment, said genetically modified malate-producing yeast strain further has phosphoenolpyruvate carboxykinase activity or enhanced phosphoenolpyruvate carboxykinase activity.

Said phosphoenolpyruvate carboxykinase activity can be generated or enhanced by expressing or over-expressing a gene encoding phosphoenolpyruvate carboxykinase. In one embodiment, the gene encoding phosphoenolpyruvate carboxykinase for example *PCK* gene, is expressed or over-expressed in said genetically modified malate-producing yeast strain. In one embodiment, the phosphoenolpyruvate carboxykinase is derived from *Saccharomyces cerevisiae, Pichia kudriαvzevii* or *Escherichia coli.*

In one embodiment, the phosphoenolpyruvate carboxykinase comprises the amino acid sequence of SEQ ID NO: 6 or an amino acid sequence having at least 75%, 80%, 85%, 90%, 91%, 92%, 93%, 94%, 95%, 96%, 97%, 98%, 99% or more identity thereto and having phosphoenolpyruvate carboxykinase activity.

In one embodiment, the phosphoenolpyruvate carboxykinase comprises an amino acid sequence encoded by the sequence of SEQ ID NO: 7, or an amino acid sequence having at least 75%, 80%, 85%, 90%, 91%, 92%, 93%, 94%, 95%, 96%, 97%, 98%, 99% or more identity thereto and having phosphoenolpyruvate carboxykinase activity.

In one embodiment, the gene encoding the phosphoenolpyruvate carboxykinase encodes an amino acid sequence comprising the sequence of SEQ ID NO: 6, or encodes an amino acid sequence which has at least 75%, 80%, 85%, 90%, 91%, 92%, 93%, 94%, 95%, 96%, 97%, 98%, 99% or more identity to the sequence of SEQ ID NO: 6 and has phosphoenolpyruvate carboxykinase activity.

In one embodiment, the gene encoding the phosphoenolpyruvate carboxykinase comprises the sequence of SEQ ID NO: 7 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having phosphoenolpyruvate carboxykinase activity.

Optionally, the gene encoding the phosphoenolpyruvate carboxykinase is incorporated into the genome of the genetically modified malate-producing yeast (for example *Pichia kudriαvzevii),* for example at a position of *JEN*2-2 loci. The gene encoding the phosphoenolpyruvate carboxykinase can be placed under the control of a suitable promoter (for example a promoter (for example as set forth in SEQ ID NO: 21) of *ENO1* gene) and/or terminator (for example a terminator (for example as set forth in SEQ ID NO: 22) of *SED1* gene).

In a further embodiment, said genetically modified malate-producing yeast strain further has activity or enhanced activity of at least one of the following enzymes: (I) *Escherichia coli* phosphoenolpyruvate carboxykinase (*PCK,* Uniprot database search number: P22259); (II) phosphoenolpyruvate carboxylase (for example *Escherichia coli* phosphoenolpyruvate carboxylase, Uniprot database search number: P00864) and (III) phosphoenolpyruvate carboxykinase (loci number PK0402) of *Pichia kudriαvzevii* (for example the *Pichia kudriαvzevii* CY902 with deposit number CGMCC No.20885). The enzyme activity can be generated and enhanced by expressing or over-expressing an enzyme encoding gene.

In one embodiment, the gene encoding the *Escherichia coli* phosphoenolpyruvate carboxykinase comprises the sequence of SEQ ID NO: 7 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having phosphoenolpyruvate carboxykinase activity, and optionally is incorporated into the genome of the genetically modified malate-producing yeast (for example *Pichia kudriavzevii* ), for example at a position of *JEN*2-2 loci. The gene encoding the carboxykinase can be placed under the control of a suitable promoter (for example a promoter (for example as set forth in SEQ ID NO: 21) of *ENO1* gene) and/or terminator (for example a terminator (for example as set forth in SEQ ID NO: 22) of *SED1* gene).

In one embodiment, the phosphoenolpyruvate carboxylase is derived from plants (especially Bacillariophyta and Angiospermophyta plants) and bacteria (for example Proteobacteria), preferably *Escherichia coli, Phaeodactylum tricornutum, Sorghum bicolor, Zea mays* and rice.

In one embodiment, the gene encoding the *Escherichia coli* phosphoenolpyruvate carboxylase comprises the sequence of SEQ ID NO: 8 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having phosphoenolpyruvate carboxylase activity, and optionally is incorporated into the genome of the genetically modified malate-producing yeast (for example *Pichia kudriαvzevii*)*,* for example at a position of *JEN2-2* loci. The gene encoding the carboxylase can be placed under the control of a suitable promoter (for example a promoter (for example as set forth in SEQ ID NO: 21) of *ENO1* gene) and/or terminator (for example a terminator (for example as set forth in SEQ ID NO: 22) of *SED1* gene).

In one embodiment, the gene encoding the phosphoenolpyruvate carboxykinase of *Pichia kudriαvzevii* encodes the amino acid sequence of SEQ ID NO: 6 or an amino acid sequence having at least 75%, 80%, 85%, 90%, 91%, 92%, 93%, 94%, 95%, 96%, 97%, 98%, 99% or more identity thereto and having phosphoenolpyruvate carboxykinase activity, and optionally is incorporated into the genome of the genetically modified malate-producing yeast (for example *Pichia kudriαvzevii*)*,* for example at a position of *JEN*2-2 loci. The gene encoding the phosphoenolpyruvate carboxykinase can be placed under the control of a suitable promoter (for example a promoter (for example as set forth in SEQ ID NO: 21) of *ENO1* gene) and/or terminator (for example a terminator (for example as set forth in SEQ ID NO: 22) of *SED1* gene).

The gene to be expressed or over-expressed can be integrated at a suitable position in the strain genome, as long as such the integration does not negatively affect the growth, proliferation and/or productivity of the strain. For example, it can be integrated at the any one or more genomic positions encoding the following proteins: (i) pyruvate decarboxylase (EC 4.1.1.43), (ii) NAD-dependent glycerol-3-phosphate dehydrogenase (EC 1.1.1.8), (iii) orotidine 5'-phosphate decarboxylase (EC 4.1.1.23), (iv) monocarboxylate permease, (v) dicarboxylate transport protein, (vi) malic enzyme (EC 1.1.1.38), and (vii) a bifunctional enzyme of oxaloacetate decarboxylase and 3-hydroxy-3-methylglutarate aldolase (EC 4.1.3.17 or 4.1.1.112).

In a further embodiment, the genetically modified malate-producing yeast strain further has at least one reduced activity of or inactivated: (i) pyruvate decarboxylase (EC 4.1.1.43), (ii) NAD-dependent glycerol-3-phosphate dehydrogenase (EC 1.1.1.8), (iii) orotidine 5'-phosphate decarboxylase (EC 4.1.1.23), (iv) monocarboxylate permease, (v) dicarboxylate transport protein, (vi) malic enzyme (EC 1.1.1.38), and (vii) a bifunctional enzyme of oxaloacetate decarboxylase and 3-hydroxy-3-methylglutarate aldolase (EC 4.1.3.17 or 4.1.1.112). In one embodiment, the genetically modified malate-producing yeast strain has activity-reduced or inactivated pyruvate decarboxylase. The activity-reduced or inactivated pyruvate decarboxylase can be achieved by attenuating or inactivating the gene encoding pyruvate decarboxylase in the strain. Therefore, in one embodiment, the genetically modified malate-producing yeast strain has an attenuated or inactivated gene encoding pyruvate decarboxylase. In one embodiment, the gene encoding pyruvate decarboxylase in the genetically modified malate-producing yeast strain is knocked out.

In one embodiment, the pyruvate decarboxylase comprises the amino acid sequence of SEQ ID NO: 10 or an amino acid sequence having at least 75%, 80%, 85%, 90%, 91%, 92%, 93%, 94%, 95%, 96%, 97%, 98%, 99% or more identity thereto and having pyruvate decarboxylase activity.

In one embodiment, the gene encoding the pyruvate decarboxylase encodes a protein of SEQ ID NO: 10 or an amino acid sequence having at least 75%, 80%, 85%, 90%, 91%, 92%, 93%, 94%, 95%, 96%, 97%, 98%, 99% or more identity thereto and having pyruvate decarboxylase activity.

In a further embodiment, the genetically modified malate-producing yeast strain further has activity-reduced or inactivated NAD-dependent glycerol-3-phosphate dehydrogenase. In one embodiment, the genetically modified malate-producing yeast strain has an attenuated or inactivated gene encoding NAD-dependent glycerol-3-phosphate dehydrogenase. In one embodiment, the gene encoding NAD-dependent glycerol-3-phosphate dehydrogenase in the genetically modified malate-producing yeast strain (for example *Pichia kudriαvzevii*) is knocked out.

In one embodiment, the NAD-dependent glycerol-3-phosphate dehydrogenase comprises the amino acid sequence of SEQ ID NO: 11 or an amino acid sequence having at least 75%, 80%, 85%, 90%, 91%, 92%, 93%, 94%, 95%, 96%, 97%, 98%, 99% or more identity thereto and having NAD-dependent glycerol-3-phosphate dehydrogenase activity.

In one embodiment, the gene encoding NAD-dependent glycerol-3-phosphate dehydrogenase encodes a protein of SEQ ID NO: 11 or an amino acid sequence having at least 75%, 80%, 85%, 90%, 91%, 92%, 93%, 94%, 95%, 96%, 97%, 98%, 99% or more identity thereto and having NAD-dependent glycerol-3-phosphate dehydrogenase activity.

In a further embodiment, the genetically modified malate-producing yeast strain further has activity-reduced or inactivated orotidine 5'-phosphate decarboxylase, dicarboxylate transport protein, malic enzyme, a bifunctional enzyme of oxaloacetate decarboxylase and 3-hydroxy-3-methylglutarate aldolase, and/or monocarboxylate permease. In a further embodiment, a gene encoding orotidine 5'-phosphate decarboxylase and/or a gene encoding dicarboxylate transport protein (for example a *JEN2* gene such as *JEN2-1* gene and *JEN2-2* gene) and/or a gene encoding monocarboxylate permease and/or a gene encoding malic enzyme and/or a gene encoding a bifunctional enzyme of oxaloacetate decarboxylase and 3-hydroxy-3-methylglutarate aldolase, in the genetically modified malate-producing yeast strain is knocked out.

In one embodiment, the genetically modified malate-producing yeast strain further has activity-reduced or inactivated malic enzyme. Specifically, the genetically modified malate-producing yeast strain has an attenuated or inactivated gene encoding malic enzyme. Preferably, the gene encoding malic enzyme in the genetically modified malate-producing yeast strain (for example *Pichia kudriαvzevii*) is knocked out.

In one embodiment, the malic enzyme comprises the amino acid sequence of SEQ ID NO: 12 or an amino acid sequence having at least 75%, 80%, 85%, 90%, 91%, 92%, 93%, 94%, 95%, 96%, 97%, 98%, 99% or more identity thereto and having malic enzyme activity.

In one embodiment, the gene encoding the malic enzyme encodes a protein of SEQ ID NO: 12 or an amino acid sequence having at least 75%, 80%, 85%, 90%, 91%, 92%, 93%, 94%, 95%, 96%, 97%, 98%, 99% or more identity thereto and having malic enzyme activity.

In one embodiment, the genetically modified malate-producing yeast strain further has activity-reduced or inactivated bifunctional enzyme of oxaloacetate decarboxylase and 3-hydroxy-3-methylglutarate aldolase. Specifically, the genetically modified malate-producing yeast strain has an attenuated or inactivated gene encoding a bifunctional enzyme of oxaloacetate decarboxylase and 3-hydroxy-3-methylglutarate aldolase.

Preferably, the gene encoding a bifunctional enzyme of oxaloacetate decarboxylase and 3-hydroxy-3-methylglutarate aldolase in the genetically modified malate-producing yeast strain (for example *Pichia kudriαvzevii*) is knocked out.

In one embodiment, the bifunctional enzyme of oxaloacetate decarboxylase and 3-hydroxy-3-methylglutarate aldolase comprises the amino acid sequence of SEQ ID NO: 9 or an amino acid sequence having at least 75%, 80%, 85%, 90%, 91%, 92%, 93%, 94%, 95%, 96%, 97%, 98%, 99% or more identity thereto and having activity of a bifunctional enzyme of oxaloacetate decarboxylase and 3-hydroxy-3-methylglutarate aldolase.

In one embodiment, the gene encoding a bifunctional enzyme of oxaloacetate decarboxylase and 3-hydroxy-3-methylglutarate aldolase encodes a protein of SEQ ID NO: 9 or an amino acid sequence having at least 75%, 80%, 85%, 90%, 91%, 92%, 93%, 94%, 95%, 96%, 97%, 98%, 99% or more identity thereto and having activity of a bifunctional enzyme of oxaloacetate decarboxylase and 3-hydroxy-3-methylglutarate aldolase.

In one embodiment, the genetically modified malate-producing yeast strain further has activity-reduced or inactivated orotidine 5'-phosphate decarboxylase. Specifically, the genetically modified malate-producing yeast strain has an attenuated or inactivated gene encoding orotidine 5'-phosphate decarboxylase. Preferably, the gene encoding orotidine 5'-phosphate decarboxylase in the genetically modified malate-producing yeast strain (for example *Pichia kudriαvzevii*) is knocked out.

In one embodiment, the orotidine 5'-phosphate decarboxylase comprises the amino acid sequence of SEQ ID NO: 13 or an amino acid sequence having at least 75%, 80%, 85%, 90%, 91%, 92%, 93%, 94%, 95%, 96%, 97%, 98%, 99% or more identity thereto and having orotidine 5'-phosphate decarboxylase activity.

In one embodiment, the gene encoding the orotidine 5'-phosphate decarboxylase encodes a protein of SEQ ID NO: 13 or an amino acid sequence having at least 75%, 80%, 85%, 90%, 91%, 92%, 93%, 94%, 95%, 96%, 97%, 98%, 99% or more identity thereto and having orotidine 5'-phosphate decarboxylase activity.

In one embodiment, the genetically modified malate-producing yeast strain further has activity-reduced or inactivated monocarboxylate permease. Specifically, the genetically modified malate-producing yeast strain has an attenuated or inactivated gene encoding monocarboxylate permease. Preferably, the gene encoding monocarboxylate permease in the genetically modified malate-producing yeast strain (for example *Pichia kudriαvzevii*) is knocked out.

In one embodiment, the monocarboxylate permease comprises the amino acid sequence of SEQ ID NO: 16 or an amino acid sequence having at least 75%, 80%, 85%, 90%, 91%, 92%, 93%, 94%, 95%, 96%, 97%, 98%, 99% or more identity thereto and having monocarboxylate permease activity.

In one embodiment, the gene encoding the monocarboxylate permease encodes a protein of SEQ ID NO: 16 or an amino acid sequence having at least 75%, 80%, 85%, 90%, 91%, 92%, 93%, 94%, 95%, 96%, 97%, 98%, 99% or more identity thereto and having monocarboxylate permease activity.

In one embodiment, the genetically modified malate-producing yeast strain further has activity-reduced or inactivated dicarboxylate transport protein. Specifically, the genetically modified malate-producing yeast strain has an attenuated or inactivated gene encoding dicarboxylate transport protein. Preferably, the gene encoding dicarboxylate transport protein in the genetically modified malate-producing yeast strain (for example *Pichia kudriαvzevii*)*,* such as *JEN2-1* or *JEN2-2* gene, is knocked out.

In one embodiment, the dicarboxylate transport protein comprises the amino acid sequence of SEQ ID NO: 14 or 15 or an amino acid sequence having at least 75%, 80%, 85%, 90%, 91%, 92%, 93%, 94%, 95%, 96%, 97%, 98%, 99% or more identity thereto and having dicarboxylate transport protein activity.

In one embodiment, the gene encoding the dicarboxylate transport protein encodes a protein of SEQ ID NO: 14 or 15, or an amino acid sequence having at least 75%, 80%, 85%, 90%, 91%, 92%, 93%, 94%, 95%, 96%, 97%, 98%, 99% or more identity thereto and having dicarboxylate transport protein activity.

In one embodiment, the present invention provides a genetically modified malate-producing yeast (for example *Pichia kudriαvzevii,* such as the CY902 strain) strain, having an over-expressed gene encoding malate transport protein, such as *SpMAE1* gene, and an over-expressed *MDH* gene encoding NADPH-dependent malate dehydrogenase, and optionally wherein, an endogenous gene encoding orotidine 5'-phosphate decarboxylase and/or an endogenous gene encoding monocarboxylate permease and/or an endogenous gene encoding a bifunctional enzyme of oxaloacetate decarboxylase and 3-hydroxy-3-methylglutarate aldolase is knocked out. Preferably, the *MDH* gene comprises the sequence of SEQ ID NO: 1 or a degenerate sequence thereof, or the gene encoding malate transport protein comprises the sequence of SEQ ID NO: 2 or a degenerate sequence thereof.

In one embodiment, the present invention provides a genetically modified malate-producing yeast (for example *Pichia kudriαvzevii,* such as the CY902 strain) strain, having an over-expressed *MDH* gene encoding NADPH-dependent malate dehydrogenase and an over-expressed gene encoding malate transport protein, such as *SpMAE1* gene, wherein an endogenous gene encoding pyruvate decarboxylase and an endogenous gene encoding NAD-dependent glycerol-3-phosphate dehydrogenase are knocked out, and optionally wherein, an endogenous gene encoding orotidine 5'-phosphate decarboxylase and/or an endogenous gene encoding monocarboxylate permease and/or an endogenous gene encoding a bifunctional enzyme of oxaloacetate decarboxylase and 3-hydroxy-3-methylglutarate aldolase is knocked out. Preferably, the *MDH* gene comprises the sequence of SEQ ID NO: 1 or a degenerate sequence thereof, or the gene encoding malate transport protein comprises the sequence of SEQ ID NO: 2 or a degenerate sequence thereof.

In one embodiment, the present invention provides a genetically modified malate-producing yeast (for example *Pichia kudriαvzevii,* such as CY902 strain) strain, having an over-expressed *MDH* gene encoding NADPH-dependent malate dehydrogenase and an over-expressed gene encoding malate transport protein for example *SpMAE1* gene, wherein an endogenous gene encoding malic enzyme is knocked out, and optionally wherein, an endogenous gene encoding orotidine 5'-phosphate decarboxylase and/or an endogenous gene encoding monocarboxylate permease and/or an endogenous gene encoding a bifunctional enzyme of oxaloacetate decarboxylase and 3-hydroxy-3-methylglutarate aldolase is knocked out. Preferably, the *MDH* gene comprises the sequence of SEQ ID NO: 1 or a degenerate sequence thereof, or the gene encoding malate transport protein comprises the sequence of SEQ ID NO: 2 or a degenerate sequence thereof.

In one embodiment, the present invention provides a genetically modified malate-producing yeast (for example *Pichia kudriαvzevii,* such as the CY902 strain) strain, having an over-expressed *MDH* gene encoding NADPH-dependent malate dehydrogenase and an over-expressed gene encoding malate transport protein for example *SpMAE1* gene, wherein an endogenous gene encoding pyruvate decarboxylase, an endogenous gene encoding NAD-dependent glycerol-3-phosphate dehydrogenase and an endogenous gene encoding malic enzyme are knocked out, and optionally wherein, an endogenous gene encoding orotidine 5'-phosphate decarboxylase and/or an endogenous gene encoding monocarboxylate permease and/or an endogenous gene encoding a bifunctional enzyme of oxaloacetate decarboxylase and 3-hydroxy-3-methylglutarate aldolase is knocked out. Preferably, the *MDH* gene comprises the sequence of SEQ ID NO: 1 or a degenerate sequence thereof, or the gene encoding malate transport protein comprises the sequence of SEQ ID NO: 2 or a degenerate sequence thereof.

In one embodiment, the present invention provides a genetically modified malate-producing yeast (for example *Pichia kudriαvzevii*) strain, having over-expressed genes as follows:
an *MDH* gene encoding NADPH-dependent malate dehydrogenase,
a gene encoding malate transport protein for example *SpMAE1* gene, and
at least one of the following genes: a gene encoding biotin transport protein for example *SpVHT1* gene,
a gene encoding *Escherichia coli* phosphoenolpyruvate carboxykinase, a gene encoding phosphoenolpyruvate carboxylase preferably *Escherichia coli* phosphoenolpyruvate carboxylase, a gene encoding *Pichia kudriαvzevii* phosphoenolpyruvate carboxykinase and a gene encoding pyruvate carboxylase,

optionally wherein, an endogenous gene encoding pyruvate decarboxylase and an endogenous gene encoding NAD-dependent glycerol-3-phosphate dehydrogenase in the strain are knocked out,
optionally wherein, an endogenous gene encoding orotidine 5'-phosphate decarboxylase and/or an endogenous gene encoding monocarboxylate permease and/or an endogenous gene encoding dicarboxylate transport protein for example *JEN2-1* or *JEN*2-2 gene and/or an endogenous gene encoding a bifunctional enzyme of oxaloacetate decarboxylase and 3-hydroxy-3-methylglutarate aldolase and/or an endogenous gene encoding malic enzyme is knocked out.

In one embodiment, the present invention provides a genetically modified malate-producing yeast (for example *Pichia kudriαvzevii*) strain, having an over-expressed *MDH* gene encoding NADPH-dependent malate dehydrogenase, an over-expressed gene encoding malate transport protein for example *SpMAE1* gene and an over-expressed gene encoding biotin transport protein for example *SpVHT1* gene, and optionally wherein, an endogenous gene encoding orotidine 5'-phosphate decarboxylase and/or an endogenous gene encoding monocarboxylate permease and/or an endogenous gene encoding a bifunctional enzyme of oxaloacetate decarboxylase and 3-hydroxy-3-methylglutarate aldolase and/or an endogenous gene encoding malic enzyme is knocked out. Preferably, the *MDH* gene comprises the sequence of SEQ ID NO: 1 or a degenerate sequence thereof, the gene encoding malate transport protein comprises the sequence of SEQ ID NO: 2 or a degenerate sequence thereof, or the gene encoding biotin transport protein comprises the sequence of SEQ ID NO: 3 or a degenerate sequence thereof.

In one embodiment, the present invention provides a genetically modified malate-producing yeast (for example *Pichia kudriαvzevii,* such as the CY902) strain, having an over-expressed *MDH* gene encoding NADPH-dependent malate dehydrogenase, an over-expressed gene encoding malate transport protein for example *SpMAE1* gene, and an over-expressed gene encoding biotin transport protein for example *SpVHT1* gene, wherein an endogenous gene encoding malic enzyme for example *PkMAE1* gene is knocked out, and optionally wherein, an endogenous gene encoding orotidine 5'-phosphate decarboxylase for example *URA3* gene, and/or an endogenous gene encoding monocarboxylate permease for example *MCH4* gene, and/or an endogenous gene encoding a bifunctional enzyme of oxaloacetate decarboxylase and 3-hydroxy-3-methylglutarate aldolase for example *Pk2365* gene is knocked out. Preferably, the *MDH* gene comprises the sequence of SEQ ID NO: 1 or a degenerate sequence thereof, the gene encoding malate transport protein comprises the sequence of SEQ ID NO: 2 or a degenerate sequence thereof, or the gene encoding biotin transport protein comprises the sequence of SEQ ID NO: 3 or a degenerate sequence thereof.

In one embodiment, the present invention provides a genetically modified malate-producing yeast strain (for example the CY902 strain), having an over-expressed *MDH* gene encoding NADPH-dependent malate dehydrogenase and an over-expressed gene encoding malate transport protein, as well as at least one over-expressed gene of the followings:
a gene encoding biotin transport protein, a gene encoding *Escherichia coli* phosphoenolpyruvate carboxykinase, a gene encoding phosphoenolpyruvate carboxylase preferably a gene encoding *Escherichia coli* phosphoenolpyruvate carboxylase, a gene encoding *Pichia kudriαvzevii* phosphoenolpyruvate carboxykinase and a gene encoding pyruvate carboxylase,
optionally wherein, an endogenous *PDC1* gene and an endogenous *GPD1* gene is knocked out,
optionally wherein, an endogenous *URA3* gene and/or an endogenous *MCH4* gene and/or an endogenous *JEN2-1* and/or *JEN2-2* gene and/or an endogenous *PkMAE* gene in the strain is knocked out.

Preferably, the *MDH* gene comprises the sequence of SEQ ID NO: 1 or a degenerate sequence thereof, the gene encoding malate transport protein comprises the sequence of SEQ ID NO: 2 or a degenerate sequence thereof, the gene encoding biotin transport protein comprises the sequence of SEQ ID NO: 3 or a degenerate sequence thereof, the gene encoding *Escherichia coli* phosphoenolpyruvate carboxykinase comprises the sequence of SEQ ID NO: 7 or a degenerate sequence thereof, the gene encoding phosphoenolpyruvate carboxylase comprises the sequence of SEQ ID NO: 8 or a degenerate sequence thereof, the gene encoding *Pichia kudriαvzevii* phosphoenolpyruvate carboxykinase encodes the sequence of SEQ ID NO: 6, or the gene encoding pyruvate carboxylase comprises the sequence of SEQ ID NO:4 or a degenerate sequence thereof.

In one embodiment, the present invention provides a genetically modified malate-producing yeast strain (for example CY902 strain), having over-expressed genes as follows: an *MDH* gene encoding NADPH-dependent malate dehydrogenase; a gene encoding malate transport protein; a gene encoding *Escherichia coli* phosphoenolpyruvate carboxylase; a gene encoding biotin transport protein; and a gene encoding *Aspergillus oryzae* pyruvate carboxylase, and wherein an endogenous gene encoding pyruvate decarboxylase and/or an endogenous gene encoding NAD-dependent glycerol-3-phosphate dehydrogenase is knocked out, and optionally wherein, an endogenous *URA3* gene and/or an endogenous *MCH4* gene and/or an endogenous *JEN2-1* and/or *JEN2-2* gene and/or an endogenous *PkMAE1* gene and/or an endogenous *Pk2365* in the strain is knocked out.

In one aspect, the present invention provides a method for preparing a genetically modified malate-producing yeast strain, comprising conferring on the strain the activity of or enhancing in the strain the activity of malate transport protein for example SpMAE1 protein, and conferring on the strain the activity of or enhancing in the strain the activity of NADPH-dependent malate dehydrogenase (EC 1.1.1.82), optionally further comprising conferring or enhancing the activity of at least one of: (i) pyruvate carboxylase (EC 6.4.1.1) activity, (ii) phosphoenolpyruvate carboxykinase (EC 4.1.1.49) activity, (iii) phosphoenolpyruvate carboxylase activity, preferably *Escherichia coli* phosphoenolpyruvate carboxylase activity, and (iv) biotin transport protein activity.

As used herein, "conferring ...activity" refers to generating an activity in the genetically modified malate-producing yeast strain, which was not present in the initial strain prior to genetic modification.

As used herein, "enhancing...activity" refers to increasing the activity for example by at least 5%, at least 10%, at least 20%, at least 30%, at least 40%, at least 50%, at least 60%, at least 70%, at least 80%, at least 90%, at least 95%, at least 100%, at least 150%, at least 200%, at least 250%, at least 300% or more.

There are many methods known in art for conferring or enhancing the desired protein activity, for example including but not limited to expressing or over-expressing a protein-encoding gene as well as the mutation or other modification that increases the protein activity.

As used herein, "over-expressing" means that the gene expression level is increased for example by at least 5%, at least 10%, at least 20%, at least 30%, at least 40%, at least 50%, at least 60%, at least 70%, at least 80%, at least 90%, at least 95%, at least 100%, at least 150%, at least 200%, at least 250%, at least 300% or more, compared to the level prior to genetic modification. Methods for over-expressing a gene are well-known in the art, for example including but not limited to using a strong promoter, increasing gene copy number, an enhancer, etc. The increased gene copy number can be achieved by for example, but not limited to, introducing one or more copies of an exogenous gene or an endogenous gene, for example through an expression vector or by integrating into a genome.

As used herein, the term "exogenous gene" refers to a gene from another cell or organism, for example a gene from the same species or a different species.

As used herein, the term "endogenous gene" refers to a gene of a cell or organism itself.

The promoter can be selected from the group consisting of any suitable promoters well-known in the art, for example including, but not limited to, a promoter of *FBA1* gene encoding fructose 1,6-bisphosphate aldolase, a promoter of *TDH3* gene encoding glyceraldehyde-3-phosphate dehydrogenase, a promoter of *PDC1* gene encoding pyruvate decarboxylase, a promoter of *ADH1* gene encoding alcohol dehydrogenase, a promoter of *PGK1* gene encoding 3-phosphoglycerate kinase, a promoter of *TEF1* gene encoding a transcriptional elongation factor, a promoter of *GPM1* gene encoding phosphoglycerate mutase, a promoter of *TPI1* gene encoding triose phosphate isomerase and a promoter of an *ENO1* gene encoding enolase.

In one embodiment, the NADPH-dependent malate dehydrogenase is derived from a plant, preferably a C4 plant, more preferably a plant of the family Gramineae, Cyperaceae, Compositae, Euphorbiaceae, Chenopodiaceae, Portulacaceae and Amaranthaceae, or is derived from *Euglenα* and *Thermobacillus,* more preferably from *Sorghum bicolor, Zea mays, Saccharum officinαrum, Pisum sativum, Cicer arietinum, Spinαciα oleracea, Euglenα gracilis* or *Methanothermobacter thermautotrophicus.*

In one embodiment, the NADPH-dependent malate dehydrogenase activity is generated or increased by expressing or over-expressing an *MDH* gene encoding NADPH-dependent malate dehydrogenase in the genetically modified malate-producing yeast strain. Preferably, the *MDH* gene encoding the NADPH-dependent malate dehydrogenase is preferably derived from a plant, preferably a C4 plant, more preferably a plant of the family Gramineae, Cyperaceae, Compositae, Euphorbiaceae, Chenopodiaceae, Portulacaceae and Amaranthaceae, or is derived from *Euglenα* and *Thermobacillus,* more preferably from *Sorghum bicolor, Zea mays, Saccharum officinαrum, Pisum sativum, Cicer arietinum, Spinαciα oleracea, Euglenα gracilis* or *Methanothermobacter thermautotrophicus,* more preferably *Sorghum bicolor.*

In one embodiment, said *MDH* gene encoding the NADPH-dependent malate dehydrogenase comprises the sequence of SEQ ID NO: 1 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having NADPH-dependent malate dehydrogenase activity.

In one embodiment, said *MDH* gene encoding NADPH-dependent malate dehydrogenase is incorporated into the genome of the genetically modified malate-producing yeast (for example *Pichia kudriαvzevii*)*,* for example at a position of *Pk2365* loci. In one embodiment, said *MDH* gene encoding NADPH-dependent malate dehydrogenase is incorporated into the genome of the yeast (for example *Pichia kudriαvzevii*) via homologous recombination, for example at a position of *MDH2* loci. Said *MDH* gene encoding NADPH-dependent malate dehydrogenase can be placed under the control of a suitable promoter (for example a promoter of *FBA1* gene (for example as set forth in SEQ ID NO: 17)) and/or terminator (for example a terminator of *INO1* gene (for example as set forth in SEQ ID NO: 18)).

In one embodiment, the malate transport protein is selected from the group consisting of SpMAE1 protein, C4T318 protein and AsDct protein. In one embodiment, the malate transport protein activity is conferred or enhanced by expressing or over-expressing the gene encoding malate transport protein for example a gene encoding SpMAE1 protein (for example *SpMAE1* gene) in the genetically modified malate-producing yeast strain.

In one embodiment, said *SpMAE1* gene comprises the sequence of SEQ ID NO: 2 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having malate transport protein activity, and optionally, (for example via homologous recombination) is incorporated into the genome of the genetically modified malate-producing yeast strain (for example *Pichia kudriαvzevii*)*,* for example at a position of *MCH4* loci. Said *SpMAE1* gene can be placed under the control of a suitable promoter (for example a promoter (for example as set forth in SEQ ID NO: 19) of *TDH3* gene) and/or terminator (for example a terminator (for example as set forth in SEQ ID NO: 20) of *GAL2* gene).

In one embodiment, the method further comprises conferring biotin transport protein activity on the strain or enhancing biotin transport protein activity in the strain. The biotin transport protein is generally derived from fungi and bacteria, especially yeasts, *Pseudomonas* and *Rhizobium,* further preferably from *Saccharomyces cerevisiae, Schizosaccharomyces pombe, Candida glabrata, Kluyveromyces marxianus, Pseudomonas mutabilis* and *Sinorhizobium meliloti.*

In one embodiment, the biotin transport protein activity is conferred or enhanced by expressing or over-expressing a gene encoding biotin transport protein for example SpVHT1 protein (for example *SpVHT1* gene) in the genetically modified malate-producing yeast strain. Therefore, in one embodiment, said biotin transport protein activity is generated or enhanced by expressing or over-expressing *SpVHT1* gene.

In one embodiment, the gene encoding the biotin transport protein comprises the sequence of SEQ ID NO: 3 or a degenerate sequence thereof, or a nucleotide sequence having at least 75%, 80%, 85%, 90%, 91%, 92%, 93%, 94%, 95%, 96%, 97%, 98%, 99% or more identity thereto and encoding a polypeptide having the biotin transport protein activity, and optionally, is incorporated into the genome of the genetically modified malate-producing yeast (for example *Pichia kudriαvzevii*)*,* for example at a position of *PkMAE1* loci. Said gene can be placed under the control of a suitable promoter (for example a promoter (for example as set forth in SEQ ID NO: 19) of *TDH3* gene) and/or terminator (for example a terminator (for example as set forth in SEQ ID NO: 20) of *GAL2* gene).

In a further embodiment, the method further comprises conferring pyruvate carboxylase activity on the strain or enhancing pyruvate carboxylase activity in the yeast strain. Preferably, conferring or enhancing pyruvate carboxylase activity is achieved by expressing or over-expressing a gene encoding pyruvate carboxylase for example *PYC* gene in the strain. The pyruvate carboxylase can be derived from fungi, especially yeasts and filamentous fungi, preferably *Saccharomyces cerevisiae, Pichia kudriαvzevii, Aspergillus oryzae, Kluyveromyces marxianus,* etc. In one embodiment, the gene encoding the pyruvate carboxylase can be selected from the group consisting of *PYC* gene of *Aspergillus oryzae* and *PYC1* gene of *Pichia kudriavzevii.*

In one embodiment, said pyruvate carboxylase comprises the amino acid sequence of SEQ ID NO: 5 or an amino acid sequence having at least 75%, 80%, 85%, 90%, 91%, 92%, 93%, 94%, 95%, 96%, 97%, 98%, 99% or more identity thereto and having the pyruvate carboxylase activity.

In one embodiment, the gene encoding the pyruvate carboxylase comprises the sequence of SEQ ID NO: 4 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having pyruvate carboxylase activity.

Optionally, the gene encoding pyruvate carboxylase is incorporated into the genome of the genetically modified malate-producing yeast (for example *Pichia kudriαvzevii),* for example at a position of *JEN2-1* loci. The gene encoding pyruvate carboxylase can be placed under the control of a suitable promoter (for example a promoter (for example as set forth in SEQ ID NO: 19) of *TDH3* gene) and/or terminator (for example a terminator (for example as set forth in SEQ ID NO: 20) of *GAL2* gene).

In a further embodiment, the method further comprises conferring or enhancing phosphoenolpyruvate carboxykinase activity. In one embodiment, said phosphoenolpyruvate carboxykinase is derived from *Saccharomyces cerevisiae, Pichia kudriαvzevii* or *Escherichia coli.* In one embodiment, the method comprises expressing or over-expressing a gene encoding phosphoenolpyruvate carboxykinase. In a further embodiment, the method comprises expressing or over-expressing the gene encoding phosphoenolpyruvate carboxykinase.

In one embodiment, the phosphoenolpyruvate carboxykinase comprises the amino acid sequence of SEQ ID NO: 6 or an amino acid sequence having at least 75%, 80%, 85%, 90%, 91%, 92%, 93%, 94%, 95%, 96%, 97%, 98%, 99% or more identity thereto and having phosphoenolpyruvate carboxykinase activity.

In one embodiment, the gene encoding the phosphoenolpyruvate carboxykinase comprises the sequence of SEQ ID NO: 7 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having pyruvate carboxylase activity.

Optionally, the gene encoding the phosphoenolpyruvate carboxykinase is incorporated into the genome of the genetically modified malate-producing yeast (for example *Pichia kudriαvzevii),* for example at a position of *JEN2-2* loci. The gene encoding the phosphoenolpyruvate carboxykinase can be placed under the control of a suitable promoter (for example a promoter (for example as set forth in SEQ ID NO: 21) of *ENO1* gene) and/or terminator (for example a terminator (for example as set forth in SEQ ID NO: 22) of *SED1* gene).

In a further embodiment, the method further comprises conferring or enhancing the activity of one or more of the following enzymes: (I) *Escherichia coli* phosphoenolpyruvate carboxykinase (*PCK,* Uniprot database search number: P22259); (II) phosphoenolpyruvate carboxylase (for example *Escherichia coli* phosphoenolpyruvate carboxylase, Uniprot database search number: P00864) and (III) phosphoenolpyruvate carboxykinase (loci number PK0402) of *Pichia kudriαvzevii* (for example the *Pichia kudriαvzevii* strain CY902 deposited under CGMCC No. 20885). In one embodiment, the method comprises conferring or enhancing enzyme activity by expressing or over-expressing the gene encoding the enzyme.

In one embodiment, the gene encoding the *Escherichia coli* phosphoenolpyruvate carboxykinase comprises the sequence of SEQ ID NO: 7 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having the phosphoenolpyruvate carboxykinase activity, and optionally, is incorporated into the genome of the genetically modified malate-producing yeast (for *Pichia kudriαvzevii),* for example at a position of *JEN2-2* loci. The gene encoding the enzyme can be placed under the control of a suitable promoter (for example a promoter (for example as set forth in SEQ ID NO: 21) of *ENO1* gene) and/or terminator (for example a terminator (for example as set forth in SEQ ID NO: 22) of *SED1* gene).

The phosphoenolpyruvate carboxylase can be derived from plants (especially Bacillariophyta and Angiospermophyta plants) and bacteria (for example Proteobacteria), preferably from *Escherichia coli, Phaeodactylum tricornutum, Sorghum bicolor, Zea mays* and rice.

In one embodiment, the gene encoding the *Escherichia coli* phosphoenolpyruvate carboxylase comprises the sequence of SEQ ID NO: 8 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having phosphoenolpyruvate carboxylase activity, and optionally is incorporated into the genome of the genetically modified malate-producing yeast (for example *Pichia kudriαvzevii),* for example at a position of *JEN2-2* loci. The gene encoding said carboxylase can be placed under the control of a suitable promoter (for example a promoter (for example as set forth in SEQ ID NO: 21) of *ENO1* gene) and/or terminator (or example a terminator (for example as set forth in SEQ ID NO: 22) of *SED1* gene).

In one embodiment, the gene encoding the phosphoenolpyruvate carboxykinase of *Pichia kudriαvzevii* encodes the amino acid sequence of SEQ ID NO: 6 or an amino acid sequence having at least 75%, 80%, 85%, 90%, 91%, 92%, 93%, 94%, 95%, 96%, 97%, 98%, 99% or more identity thereto and having phosphoenolpyruvate carboxykinase activity, and optionally is incorporated into the genome of the genetically modified malate-producing yeast (for example *Pichia kudriavzevii*), for example at a position of *JEN2-2* loci. The gene encoding said enzyme can be placed under the control of a suitable promoter (for example a promoter (for example as set forth in SEQ ID NO: 21) of *ENO1* gene) and/or terminator (or example a terminator (for example as set forth in SEQ ID NO: 22) of *SED1* gene).

The method can integrate a gene to be expressed or over-expressed in the strain into a suitable position of the strain genome, as long as such integration does not negatively affect the growth, proliferation and/or productivity of the strain. For example, the method comprises integrating one or more of the above-mentioned genes into any one or more genomic positions encoding the following proteins: (i) pyruvate decarboxylase (EC 4.1.1.43), (ii) NAD-dependent glycerol-3-phosphate dehydrogenase (EC 1.1.1.8), (iii) orotidine 5'-phosphate decarboxylase (EC 4.1.1.23), (iv) monocarboxylate permease, (v) dicarboxylate transport protein, (vi) malic enzyme (EC 1.1.1.38), and (vii) a bifunctional enzyme of oxaloacetate decarboxylase and 3-hydroxy-3-methylglutarate aldolase (EC 4.1.3.17 or 4.1.1.112).

In a further embodiment, the method further comprises attenuating or inactivating at least one of the following enzymes in the strain: (i) pyruvate decarboxylase (EC 4.1.1.43), (ii) NAD-dependent glycerol-3-phosphate dehydrogenase (EC 1.1.1.8), (iii) orotidine 5'-phosphate decarboxylase (EC 4.1.1.23), (iv) monocarboxylate permease, (v) dicarboxylate transport protein, (vi) malic enzyme (EC 1.1.1.38), and (viii) a bifunctional enzyme of oxaloacetate decarboxylase and 3-hydroxy-3-methylglutarate aldolase (EC 4.1.3.17 or 4.1.1.112).

In one embodiment, the method further comprises reducing or inactivating the pyruvate carboxylase activity in the yeast strain.

As used herein, reducing or inactivating the activity of a protein such as an enzyme means that the activity of the protein is reduced by at least 5%, at least 10%, at least 20%, at least 30%, at least 40%, at least 50%, at least 60%, at least 70%, at least 80%, at least 90%, at least 95%, at least 99% or more, or even is undetectable. There are various means known in the art for the reduction or inactivation, including for example inhibition of gene expression such as knockdown (for example using small interfering RNA), use of a weak promoter (when the gene is a polypeptide-encoding gene), etc.; gene knockout or deletion of partical or complete sequence of a gene or a polypeptide; mutation of certain loca such as an encoding sequence or an active domain, in a gene or a polypeptide to reduce the gene expression or regulate the activity or express the activity of a product; and use of an antagonist or an inhibitor (for example including but not limited to, an antibody, interfering RNA, etc.)

As used herein, attenuating or inactivating a gene means that the expression level (when being used as a protein-encoding gene) or regulatory performance (when being used as a regulatory element) of the gene is reduced by at least 5%, at least 10%, at least 20%, at least 30%, at least 40%, at least 50%, at least 60%, at least 70%, at least 80%, at least 90%, at least 95%, at least 99% or more, or even is undetectable. There are various means in the art for attenuating or inactivating a gene, including, for example, inhibition of gene expression such as knockdown (for example using small interfering RNA), use of a weak promoter (when the gene is a polypeptide-encoding gene), etc.; gene knockout or deletion of partial or complete sequence of a gene; mutation of certain loca such as an encoding sequence in a gene to reduce the gene expression or regulate the activity or express the activity of a product, etc.

In one embodiment, reducing or inactivating pyruvate decarboxylase activity includes attenuating or inactivating a gene encoding pyruvate decarboxylase.

In one embodiment, attenuating or inactivating the gene encoding pyruvate decarboxylase comprises knocking out the gene encoding pyruvate decarboxylase (for example encoding the amino acid sequence of SEQ ID NO: 10 or an amino acid sequence having at least 75%, 80%, 85%, 90%, 91%, 92%, 93%, 94%, 95%, 96%, 97%, 98%, 99% or more identity thereto and having pyruvate decarboxylase activity).

In a further embodiment, the method further comprises reducing or inactivating NAD-dependent glycerol-3-phosphate dehydrogenase activity in the yeast strain.

In one embodiment, reducing or inactivating NAD-dependent glycerol-3-phosphate dehydrogenase activity comprises attenuating or inactivating the gene encoding NAD-dependent glycerol-3-phosphate dehydrogenase. In one embodiment, attenuating or inactivating the gene encoding NAD-dependent glycerol-3-phosphate dehydrogenase comprises knocking out the gene encoding NAD-dependent glycerol-3-phosphate dehydrogenase (for example encoding the amino acid sequence of SEQ ID NO: 11 or an amino acid sequence having at least 75%, 80%, 85%, 90%, 91%, 92%, 93%, 94%, 95%, 96%, 97%, 98%, 99% or more identity thereto and having NAD-dependent glycerol-3-phosphate dehydrogenase activity).

In a further embodiment, the method further comprises reducing or inactivating the activity (activities) of malic enzyme and/or a bifunctional enzyme of oxaloacetate decarboxylase and 3-hydroxy-3-methylglutarate aldolase and/or orotidine 5'-phosphate decarboxylase and/or monocarboxylate permease and/or dicarboxylate transport protein in the yeast strain. In one embodiment, the method comprises attenuating or inactivating a gene encoding orotidine 5'-phosphate decarboxylase and/or a gene encoding monocarboxylate permease in the yeast strain.

In one embodiment, the method comprises attenuating or inactivating one or more of a gene encoding malic enzyme, a gene encoding a bifunctional enzyme of oxaloacetate decarboxylase and 3-hydroxy-3-methylglutarate aldolase, a gene encoding orotidine 5'-phosphate decarboxylase, a gene encoding dicarboxylate transport protein (for example *JEN2* genes such as *JEN2-1* gene, *JEN2-2* gene) and a gene encoding monocarboxylate permease in the yeast strain.

In one embodiment, the gene encoding malic enzyme (for example encoding the amino acid sequence of SEQ ID NO: 12 or an amino acid sequence having at least 75%, 80%, 85%, 90%, 91%, 92%, 93%, 94%, 95%, 96%, 97%, 98%, 99% or more identity thereto and having malic enzyme activity) is knocked out, for example knocked out through homologous recombination.

In one embodiment, the gene encoding a bifunctional enzyme of oxaloacetate decarboxylase and 3-hydroxy-3-methylglutarate aldolase (for example encoding the amino acid sequence of SEQ ID NO: 9 or amino acid sequence having at least 75%, 80%, 85%, 90%, 91%, 92%, 93%, 94%, 95%, 96%, 97%, 98%, 99% or more identity thereto and having the activity of a bifunctional enzyme of oxaloacetate decarboxylase and 3-hydroxy-3-methylglutarate aldolase) is knocked out, for example knocked out through homologous recombination.

In one embodiment, the gene encoding orotidine 5'-phosphate decarboxylase (for example encoding the amino acid sequence of SEQ ID NO: 13 or an amino acid sequence having at least 75%, 80%, 85%, 90%, 91%, 92%, 93%, 94%, 95%, 96%, 97%, 98%, 99% or more identity thereto and having orotidine 5'-phosphate decarboxylase activity) is knocked out, for example knocked out through homologous recombination.

In one embodiment, the *JEN2-1* gene encoding dicarboxylate transport protein (for example encoding the amino acid sequence of SEQ ID NO: 14 or an amino acid sequence having at least 75%, 80%, 85%, 90%, 91%, 92%, 93%, 94%, 95%, 96%, 97%, 98%, 99% or more identity thereto and having dicarboxylate transport protein activity) is knocked out, for example knocked out through homologous recombination.

In one embodiment, the *JEN*2-2 gene encoding dicarboxylate transport protein (for example encoding the amino acid sequence of SEQ ID NO: 15 or an amino acid sequence having at least 75%, 80%, 85%, 90%, 91%, 92%, 93%, 94%, 95%, 96%, 97%, 98%, 99% or more identity thereto and having dicarboxylate transport protein activity) is knocked out, for example knocked out through homologous recombination.

In one embodiment, the gene encoding monocarboxylate permease (for example encoding the amino acid sequence of SEQ ID NO: 16 or an amino acid sequence having at least 75%, 80%, 85%, 90%, 91%, 92%, 93%, 94%, 95%, 96%, 97%, 98%, 99% or more identity thereto and having monocarboxylate permease activity) is knocked out, for example knocked out through homologous recombination.

In one embodiment, the present invention provides a method for preparing a genetically modified malate-producing yeast (for example *Pichia kudriαvzevii,* such as CY902) strain, comprising over-expressing *MDH* gene encoding NADPH-dependent malate dehydrogenase and a gene encoding malate transport protein for example *SpMAE1* gene in the genetically modified malate-producing yeast strain, and optionally comprising knocking out an endogenous gene encoding orotidine 5'-phosphate decarboxylase and/or an endogenous gene encoding monocarboxylate permease and/or an endogenous gene encoding a bifunctional enzyme of oxaloacetate decarboxylase and 3-hydroxy-3-methylglutarate aldolase. Preferably, the *MDH* gene comprises the sequence of SEQ ID NO: 1 or a degenerate sequence thereof, or the gene encoding malate transport protein comprises the sequence of SEQ ID NO: 2 or a degenerate sequence thereof.

In one embodiment, the present invention provides a method for preparing a genetically modified malate-producing yeast (for example *Pichia kudriαvzevii,* such as CY902) strain, comprising in the genetically modified malate-producing yeast strain: over-expressing *MDH* gene encoding NADPH-dependent malate dehydrogenase and a gene encoding malate transport protein for example *SpMAE1* gene, and knocking out an endogenous gene encoding pyruvate decarboxylase and an endogenous gene encoding NAD-dependent glycerol-3-phosphate dehydrogenase, and optionally knocking out an endogenous gene encoding orotidine 5'-phosphate decarboxylase and/or an endogenous gene encoding monocarboxylate permease and/or an endogenous gene encoding a bifunctional enzyme of oxaloacetate decarboxylase and 3-hydroxy-3-methylglutarate aldolase. Preferably, the *MDH* gene comprises the sequence of SEQ ID NO: 1 or a degenerate sequence thereof, or the gene encoding malate transport protein comprises the sequence of SEQ ID NO: 2 or a degenerate sequence thereof.

In one embodiment, the present invention provides a method for preparing a genetically modified malate-producing yeast (for example *Pichia kudriαvzevii,* such as CY902) strain, comprising in the genetically modified malate-producing yeast strain: over-expressing *MDH* gene encoding NADPH-dependent malate dehydrogenase and a gene encoding malate transport protein for example *SpMAE1* gene, and knocking out an endogenous gene encoding malic enzyme, and optionally knocking out an endogenous gene encoding orotidine 5'-phosphate decarboxylase and/or an endogenous gene encoding monocarboxylate permease and/or an endogenous gene encoding a bifunctional enzyme of oxaloacetate decarboxylase and 3-hydroxy-3-methylglutarate aldolase. Preferably, the *MDH* gene comprises the sequence of SEQ ID NO: 1 or a degenerate sequence thereof, or the gene encoding malate transport protein comprises the sequence of SEQ ID NO: 2 or a degenerate sequence thereof.

In one embodiment, the present invention provides a method for preparing a genetically modified malate-producing yeast (for example *Pichia kudriαvzevii,* such as CY902) strain, comprising in the genetically modified malate-producing yeast strain: over-expressing *MDH* gene encoding NADPH-dependent malate dehydrogenase and a gene encoding malate transport protein for example *SpMAE1* gene, and knocking out an endogenous gene encoding pyruvate decarboxylase, an endogenous gene encoding NAD-dependent glycerol-3-phosphate dehydrogenase and an endogenous gene encoding malic enzyme, and optionally knocking out an endogenous gene encoding orotidine 5'-phosphate decarboxylase and/or an endogenous gene encoding monocarboxylate permease and/or an endogenous gene encoding a bifunctional enzyme of oxaloacetate decarboxylase and 3-hydroxy-3-methylglutarate aldolase. Preferably, the *MDH* gene comprises the sequence of SEQ ID NO: 1 or a degenerate sequence thereof, or the gene encoding malate transport protein comprises the sequence of SEQ ID NO: 2 or a degenerate sequence thereof.

In one embodiment, the present invention provides a method for preparing a genetically modified malate-producing yeast (for example *Pichia kudriαvzevii,* such as CY902) strain, comprising over-expressing the following genes in the genetically modified malate-producing yeast strain:
*MDH* gene encoding NADPH-dependent malate dehydrogenase,
a gene encoding malate transport protein for example *SpMAE1* gene, and
at least one of the following genes: a gene encoding biotin transport protein, a gene encoding *Escherichia coli* phosphoenolpyruvate carboxykinase, a gene encoding phosphoenolpyruvate carboxylase preferably *Escherichia coli* phosphoenolpyruvate carboxylase, a gene encoding *Pichia kudriαvzevii* phosphoenolpyruvate carboxykinase and a gene encoding pyruvate carboxylase, and

optionally, knocking out an endogenous gene encoding pyruvate decarboxylase and/or an endogenous gene encoding NAD-dependent glycerol-3-phosphate dehydrogenase,
optionally, knocking out an endogenous gene encoding orotidine 5'-phosphate decarboxylase and/or an endogenous gene encoding monocarboxylate permease and/or an endogenous gene encoding dicarboxylate transport protein, such as *JEN2-1* or *JEN2-2* gene, and/or an endogenous gene encoding a bifunctional enzyme of oxaloacetate decarboxylase and 3-hydroxy-3-methylglutarate aldolase and/or an endogenous gene encoding malic enzyme.

In one embodiment, the present invention provides a method for preparing a genetically modified malate-producing yeast (for example *Pichia kudriαvzevii,* such as CY902) strain, comprising in the genetically modified malate-producing yeast strain: over-expressing *MDH* gene encoding NADPH-dependent malate dehydrogenase and a gene encoding malate transport protein for example *SpMAE1* gene and a gene encoding biotin transport protein for example *SpVHT1* gene, and optionally knocking out an endogenous gene encoding orotidine 5'-phosphate decarboxylase and/or an endogenous gene encoding monocarboxylate permease and/or an endogenous gene encoding a bifunctional enzyme of oxaloacetate decarboxylase and 3-hydroxy-3-methylglutarate aldolase and/or an endogenous gene encoding malic enzyme. Preferably, the *MDH* gene comprises the sequence of SEQ ID NO: 1 or a degenerate sequence thereof, the gene encoding malate transport protein comprises the sequence of SEQ ID NO: 2 or a degenerate sequence thereof, or the gene encoding biotin transport protein comprises the sequence of SEQ ID NO: 3 or a degenerate sequence thereof.

In one embodiment, the present invention provides a method for preparing a genetically modified malate-producing yeast (for example *Pichia kudriαvzevii,* such as CY902) strain, comprising in the genetically modified malate-producing yeast strain: over-expressing *MDH* gene encoding NADPH-dependent malate dehydrogenase, a gene encoding malate transport protein for example *SpMAE1* gene and a gene encoding biotin transport protein for example *SpVHT1* gene, and knocking out an endogenous gene encoding malic enzyme, and optionally knocking out an endogenous gene encoding orotidine 5'-phosphate decarboxylase and/or an endogenous gene encoding monocarboxylate permease and/or an endogenous gene encoding a bifunctional enzyme of oxaloacetate decarboxylase and 3-hydroxy-3-methylglutarate aldolase. Preferably, the *MDH* gene comprises the sequence of SEQ ID NO: 1 or a degenerate sequence thereof, the gene encoding malate transport protein comprises the sequence of SEQ ID NO: 2 or a degenerate sequence thereof, or the gene encoding biotin transport protein comprises the sequence of SEQ ID NO: 3 or a degenerate sequence thereof.

In one embodiment, the malate-producing yeast to be genetically modified includes, for example, but is not limited to the genera *Candida, Pichia, Rhodotroula, Saccharomyces, Yarrowia, Zygosaccharomyces* and *Torulopsis.* In one embodiment, the malate-producing yeast to be genetically modified is selected from the group consisting of the genus *Pichia.* In one preferred embodiment, the malate-producing yeast to be genetically modified is *Pichia kudriαvzevii,* for example the *Pichia kudriαvzevii* deposited in China General Microbiological Culture Collection Center (CGMCC) o under the deposit number of CGMCC No. 20885.

In one embodiment, the present invention provides a method for preparing a genetically modified *Pichia kudriαvzevii* strain (such as CY902 strain), comprising in the strain: over-expressing *MDH* gene encoding NADPH-dependent malate dehydrogenase and a gene encoding malate transport protein, and optionally knocking out endogenous *URA3* gene and/or endogenous *MCH4* gene in the strain, wherein preferably the *MDH* gene comprises the sequence of SEQ ID NO: 1 or a degenerate sequence thereof, or the gene encoding malate transport protein comprises the sequence of SEQ ID NO: 2 or a degenerate sequence thereof.

In one embodiment, the present invention provides a method for preparing a genetically modified *Pichia kudriαvzevii* strain (for example CY902 strain), comprising in the strain: over-expressing *MDH* gene encoding NADPH-dependent malate dehydrogenase and a gene encoding malate transport protein, and knocking out endogenous *PDC1* gene and/or endogenous *GPD1* gene in the strain, and optionally knocking out endogenous *URA3* gene and/or endogenous *MCH4* gene and/or endogenous *JEN2-1* and/or *JEN2-2* gene and/or endogenous *PkMAE* gene. Preferably, the *MDH* gene comprises the sequence of SEQ ID NO: 1 or a degenerate sequence thereof, or the gene encoding malate transport protein comprises the sequence of SEQ ID NO: 2 or a degenerate sequence thereof.

In one embodiment, the present invention provides a method for preparing a genetically modified *Pichia kudriαvzevii* strain (for example CY902 strain), comprising in the strain over-expressing:
*MDH* gene encoding NADPH-dependent malate dehydrogenase;
a gene encoding malate transport protein;
a gene encoding *Escherichia coli* phosphoenolpyruvate carboxylase;
a gene encoding biotin transport protein; and
a gene encoding *Aspergillus oryzae* pyruvate carboxylase, and

knocking out an endogenous gene encoding pyruvate decarboxylase and/or an endogenous gene encoding NAD-dependent glycerol-3-phosphate dehydrogenase,
optionally, the endogenous *URA3* gene and/or the endogenous *MCH4* gene and/or the endogenous *JEN2-1* and/or *JEN*2-2 gene and/or the endogenous *PkMAE1* gene and/or the endogenous *Pk2365* gene in the strain being knocked out.

In one aspect, the present invention provides a method for producing L-malate, comprising culturing the genetically modified malate-producing yeast strain of the present invention or a genetically modified malate-producing yeast strain prepared by the method for preparing a genetically modified malate-producing yeast strain according to the present invention under suitable conditions for the fermentation production of L-malate, optionally comprising isolating and purifying the produced L-malate.

It is known in the art that conditions for the fermentation production of L-malate, under which the malate-producing yeast strain is cultured through fermentation, include for example but are not limited to pH, temperature, culture medium components, fermentation time, etc.

It is known in the art that the culture medium for the fermentation production of L-malate by the malate-producing yeast strain includes for example but is not limited to an inorganic salt culture medium (about 5-12% w/v glucose, optionally containing about 30 g/L CaCOs).

It is known in the art that the temperature for the fermentation production of L-malate by the malate-producing yeast strain is for example about 25-37°C, for example about 25°C, about 26°C, about 27°C, about 28°C, about 29°C, about 30°C, about 31°C, about 32°C, about 33°C, about 34°C, about 35°C, about 36°C or about 37°C. In one embodiment, the malate-producing yeast strain of the present invention is fermented at 30°C to produce L-malate.

The malate-producing yeast strain of the present invention can be fermented at a suitable pH known in the art, for example at the pH value of less than about 7.0, less than about 6.5, less than about 6.0, less than about 5.5, less than about 5.0, less than about 4.5, less than about 4.0, less than about 3.5, less than about 3.0, less than about 2.5, less than about 2.0, less than about 1.5, less than about 1.0 (for example a pH value of about 1.0-7.0, 1.0-6.0, 1.0-5.5, 1.0-5.0, 1.0-4.5, 1.0-4.0, 1.0-3.5, 1.0-3.0, 2.0-7.0, 2.0-6.0, 2.0-5.5, 2.0-5.0, 2.0-4.5, 2.0-4.0, 2.0-3.5, 2.0-3.0, 3.0-7.0, 3.0-6.0, 3.0-5.5, 3.0-5.0, 3.0-4.5, 3.0-4.0, 3.0-3.5, 4.0-7.0, 4.0-6.0, 4.0-5.5, 4.0-5.0, 4.0-4.5). In one embodiment, the malate-producing yeast strain of the present invention may be fermented at the pH value of <about 3.5 to produce L-malate.

In order to produce L-malate, the malate-producing yeast strain of the present invention can be fermented for a suitable time, for example about 12-96 hours, such as about 12 hours, about 24 hours, about 36 hours, about 48 hours, about 60 hours, about 72 hours, or about 96 hours. In one embodiment, in order to produce L-malate, the malate-producing yeast strain of the present invention may be fermented for about 24-72 hours, for example about 30 hours.

The malate-producing yeast strain of the present invention can be fermented under the condition of shaking (for example at about 100-300 rpm, such as about 150, about 200 and about 250rpm) to produce L-malate.

The content of L-malate in a fermentation broth can be determined by any suitable method known in the art, for example high performance liquid chromatography (HPLC).

In one embodiment, the present invention provides a method for producing L-malate, comprising culturing the genetically modified malate-producing yeast strain in an inorganic salt culture medium (for example containing about 5% w/v glucose and about 30 g/L CaCO₃, or containing about 12% w/v glucose) under the condition of acidic pH (less than about 7.0, less than about 6.5, less than about 6.0, less than about 5.5, less than about 5.0, less than about 4.5, less than about 4.0, less than about 3.5, less than about 3.0, less than about 2.5, less than about 2.0, less than about 1.5, less than about 1.0, for example pH of about 1.0-7.0, 1.0-6.0, 1.0-5.5, 1.0-5.0, 1.0-4.5, 1.0-4.0, 1.0-3.5, 1.0-3.0, 2.0-7.0, 2.0-6.0, 2.0-5.5, 2.0-5.0, 2.0-4.5, 2.0-4.0, 2.0-3.5, 2.0-3.0, 3.0-7.0, 3.0-6.0, 3.0-5.5, 3.0-5.0, 3.0-4.5, 3.0-4.0, 3.0-3.5, 4.0-7.0, 4.0-6.0, 4.0-5.5, 4.0-5.0, 4.0-4.5), optionally comprising isolating and purifying the produced L-malate.

In one embodiment, the addition of a neutralizing agent is not required in the method for producing L-malate of the present invention.

In one aspect, the present invention provides use of the genetically modified malate-producing yeast strain of the present invention or a genetically modified malate-producing yeast strain prepared by the method for preparing a genetically modified malate-producing yeast strain according to the present invention in the production of L-malate, especially in the production of L-malate under the condition of the acidic pH (less than about 7.0, less than about 6.5, less than about 6.0, less than about 5.5, less than about 5.0, less than about 4.5, less than about 4.0, less than about 3.5, less than about 3.0, less than about 2.5, less than about 2.0, less than about 1.5, less than about 1.0, for example pH of about 1.0-7.0, 1.0-6.0, 1.0-5.5, 1.0-5.0, 1.0-4.5, 1.0-4.0, 1.0-3.5, 1.0-3.0, 2.0-7.0, 2.0-6.0, 2.0-5.5, 2.0-5.0, 2.0-4.5, 2.0-4.0, 2.0-3.5, 2.0-3.0, 3.0-7.0, 3.0-6.0, 3.0-5.5, 3.0-5.0, 3.0-4.5, 3.0-4.0, 3.0-3.5, 4.0-7.0, 4.0-6.0, 4.0-5.5, 4.0-5.0, 4.0-4.5) and/or without the addition of a neutralizing agent.

As used herein, "optional" or "optionally" means that the subsequent described event or situation occurs or does not occur, and this description includes case where the event or situation described therein occurs or does not occur. For example, the optionally comprised step refers to the presence or absence of that step.

As used herein, the term "about" refers to a numerical range including specific values, which can be reasonably considered similar to the specific values by those skilled in the art. In some embodiments, the term "about" refers to the range within standard error using measurement commonly accepted in the art. In some embodiments, the term "about" refers to +/-10% of a specific value.

The range disclosed herein should be considered to also specifically disclose all possible sub-ranges and individual values within that range. For example, the description of a range from 1 to 6 should be considered to clearly disclose sub-ranges such as 1 to 3, 1 to 4, 1 to 5, 2 to 4, 2 to 6, and 3 to 6, as well as individual numbers within that range, such as 1, 2, 3, 4, 5 and 6. This applies regardless of the breadth of the scope.

The present invention will be further illustrated through the following non-limiting examples. It is well known to those skilled in the art that many modifications can be made to the present invention without departing from the spirit of the present application, and such modifications also fall within the scope of the present invention.

Unless otherwise specified, the following experimental methods are all conventional methods. The experimental materials used can be easily obtained from commercial companies.

### Example 1: overexpression of SpMAE1 gene and NADPH-dependent MDH gene

*ΔURA3* mutant was obtained by knocking out *URA3* gene (at locus PK2075 of CY902, encoding the amino acid sequence of SEQ ID NO: 13) encoding orotidine 5'-phosphate decarboxylase through homologous recombination (Xi, Y; Zhan, T.; Xu, H.; Chen, J.; Bi, C.; Fan, F.; Zhang, X., Characterization of JEN family carboxylate transporters from the acid-tolerant yeast Pichia kudriαvzevii and their applications in succinate production. Microb Biotechnol 2021. 0(0), 1-18. doi:10.1111/1751-7915.13781). Subsequently, a CRISPR/Cas9 plasmid pWSPK-Cas9 (GenBank accession number: MW296878.1) suitable for this strain was constructed. This plasmid contains the *URA3* screening marker, and a positive transformant can be obtained by auxotrophic screening.

By using CY902 *ΔURA3* as a starting strain, *Schizosaccharomyces pombe*-derived SpMAE1 transport protein (Uniprot database search number: P50537) and *Sorghum bicolor*-derived malate dehydrogenase SbMDH (Uniprot database key number: P17606) were over-expressed in sequence. The *SpMAE1* gene (SEQ ID NO: 2) and the *SbMDH* gene (SEQ ID NO: 1) were synthesized by Nanjing GenScript Biotech Co., Ltd. and optimized according to CY902 codon preference. The *SpMAE1* gene was integrated at the *MCH4* locus of the CY902 genome. The promoter and terminator used were a promoter of glyceraldehyde-3-phosphate dehydrogenase 3 gene *TDH3* and a terminator of galactose permease gene *GAL2* of CY902 itself, respectively. The *SbMDH* gene was integrated at the site of *Pk2365* loci of the CY902 genome. The promoter and terminator used were respectively a promoter of fructose-1,6-bisphosphate aldolase gene *FBA1* and a terminator of inositol-3-phosphate synthase gene *INO1* of CY902 itself. The specific construction method was as follows:

### 1. Construction of donor DNA fragments for homologous recombination

Using CY902 genome DNA as a template, An upstream homologous arm fragment 1 (Fragment 1) of *MCH4* gene was amplified with primers 1_UP_MCH4_F and 1_UP_MCH4_R1 (see Table 1); amplification system and procedures were based on the product specification of TAKARA PrimeSTAR^{®}HS DNA polymerase; *TDH3* gene promoter sequence (Fragment 2) of CY902 itself was amplified with primers 2_P_{TDH3}_F and 2_P_{TDH3}_R (see Table 1); Using a plasmid containing *SpMAE1* synthetic sequence as a template, SpMAE1-encoding sequence (Fragment 3, SEQ ID NO: 2) of *Schizosaccharomyces pombe* was amplified with primers 3_SpMAE1_F and 3_SpMAE1_R (see Table 1); Using CY902 genome DNA as a template, *GAL2* gene terminator sequence (Fragment 4) of CY902 itself was amplified with primers 4_T_{GAL2}_F and 4_T_{GAL2}_R (see Table 1); a downstream homologous arm fragment 1 (Fragment 5) of *MCH4* gene was amplified with primers 5_DW_MCH4_F1 and 5_DW_MCH4_R (see Table 1). Using CY902 genome DNA as a template, an upstream homologous arm fragment 2 (Fragment 6) of *MCH4* gene was amplified with primers 1_UP_MCH4_F and 1_UP_MCH4_R2 (see Table 1); a downstream homologous arm fragment 2 (Fragment 7) of *MCH4* gene was amplified with primers 5_DW_MCH4_F2 and 5_DW_MCH4_R (see Table 1).

Using CY902 genome DNA as a template, an upstream homologous arm fragment 1 (Fragment 8) of *Pk2365* gene of CY902 itself was amplified with primers 1_UP_2365_F and 1_UP_2365_R1 (see Table 1); *FBA1* promoter sequence (Fragment 9) of CY902 itself was amplified with primers 2_P_{FBA1}_F and 2_P_{FBA1_}R (see Table 1); using a plasmid containing *SbMDH* synthetic sequence as a template, SbMDH-encoding sequence (Fragment 10, SEQ ID NO:1) of *Sorghum bicolor* was amplified with primers 3_SbMDH_F and 3_SbMDH_R (see Table 1); using CY902 genome DNA as a template, *INO1* gene terminator sequence (Fragment 11) of CY902 itself was amplified with primers 4_T_{INO1_}F and 4_T_{INO1}_R (see Table 2); a downstream homologous arm fragment 1 (Fragment 12) of *Pk2365* gene of CY902 itself was amplified with primers 5_DW_2365_F1 and 5_DW_2365_R (see Table 1). Using CY902 genome DNA as a template, an upstream homologous arm fragment 2 (Fragment 13) of *Pk2365* gene of CY902 itself was amplified with primers 1_UP_2365_F and 1_UP_2365_R2 (see Table 1); a downstream homologous arm fragment 2 (Fragment 14) of *Pk2365* gene of CY902 itself was amplified with primers 5_DW_2365_F2 and 5_DW_2365_R (see Table 1).

### 2. Construction of plasmids for editing MCH4 and Pk2365 sites of CY902 itself

Using pWSPK-Cas9 plasmid (GenBank accession number: MW296878.1) as a template, a 9052 bp plasmid backbone (abbreviated as pWSPK_backbone (SEQ ID NO: 23)) without a sgRNA sequence was amplified with primers pWSPK-F and pWSPK-R (see Table 1); the 5'-end sequence (abbreviated as MCH4_sgRNA_1) of sgRNA (GenBank accession number: MW296878.1) was amplified with primers sgRNA-1F and MCH4_sgRNA-1R (see Table 1), and the 3' terminal of this fragment contained a 20 nt protospacer specific to *MCH4* gene. Using a pWSPK-Cas9 plasmid as a template, the 3'-end sequence (abbreviated as sgRNA_2, SEQ ID NO: 103) of 500 bp sgRNA was amplified with primers sgRNA-2F and sgRNA-2R (see Table 1). The above plasmid backbone pWSPK_backbone and sgRNA fragments MCH4_sgRNA_1 and sgRNA_2 were ligated by using a seamless cloning kit (Biyuntian Biotechnology Co., Ltd., Shanghai, Product Number: D7010S). The seamless cloning ligated product was transferred into Trans1-T1 competent cells (TransGen Biotech Co., Ltd., Beijing, product number: CD501-02), and the obtained positive plasmid was named pWSPK_MCH4. Using pWSPK-Cas9 plasmid as a template, the 5'-end sequence (abbreviated as 2365_sgRNA_1) of sgRNA was amplified with primers sgRNA-1F and 2365_sgRNA-1R (see Table 1), and the 3' terminal of this fragment contained a 20 nt protospacer specific to *Pk2365* gene. The pWSPK_backbone, sgRNA_2 and 2365_sgRNA_1 were ligated by using a seamless cloning kit to finally obtain the positive clone plasmid which was named pWSPK_2365.

**Table 1. Primers required for overexpression of SpMAE1 and SbMDH genes and construction of CRISPR/Cas9 plasmids**

| Primer Name | Sequence (5'-3'), protospacer underlined | SEQ ID NO |
|---|---|---|
| 1_UP_MCH4_F | AACTTTGTTGTACATTAAGCGAGTG | 24 |
| 1_UP_MCH4_R1 | GAAGTGATGTAGATACGCCAACAGGAGCAGTTGAT AATGGAAAGGGA | 25 |
| 1_UP_MCH4_R2 | AGCAGTTGATAATGGAAAGGGATAGG | 26 |
| 2_P_{TDH3}_F | CCTGTTGGCGTATCTACATCACTTC | 27 |
| 2_P_{TDH3}_R | TTTTTGTAATTGTGTTTGTTTGTGTGTTTTG | 28 |
| 3_SpMAE1_F | CAAAACACACAAACAAACACAATTACAAAAAATGG GTGAATTAAAGGAAATCTTAAAGC | 29 |
| 3_SpMAE1_R | ATTAAGACAATTGACAGAAAGTGATTTAGACGGATT CGTGTTCAGAGGA | 30 |
| 4_T_{GAL2}_F | ATCACTTTCTGTCAATTGTCTTAAT | 31 |
| 4_T_{GAL2}_R | GCGATACCGATTTAGCTAGTATTC | 32 |
| 5_DW_MCH4_F1 | GAATACTAGCTAAATCGGTATCGCTGTGTCATTGTTT GTATGGTTTAG | 33 |
| 5_DW_MCH4_F2 | CTATCCCTTTCCATTATCAACTGCTTGTCATTGTTTGT ATGGTTTAGGACT | 34 |
| 5_DW_MCH4_R | GTCAACAAATCTGACATCCAATATC | 35 |
| pWSPK-F | CTTACCAGAATAATATGCAGTTTGCT | 36 |
| pWSPK-R | GATCTCAACAGCGGTAAGATCCTTG | 37 |
| sgRNA-1F | CAAGGATCTTACCGCTGTTGAGATC | 38 |
| MCH4_sgRNA-1R | AACTTGCTATTTCTAGCTCTAAAACTGGCTACTTCT GCGGTAACATGCGCAAGCCCGGAATCGAA | 39 |
| sgRNA-2F | GTTTTAGAGCTAGAAATAGCAAGTTAAAATAAGG | 40 |
| sgRNA-2R | AGCAAACTGCATATTATTCTGGTAAG | 41 |
| 1_UP_2365_F | GGTGTCCACTCATATACTTCATTTTC | 42 |
| 1_UP_2365_R1 | ACAATACACATACAATATGGCATCGCTGAATACTAT CGAATCTTGCCAAC | 43 |
| 1_UP_2365_R2 | CTGAATACTATCGAATCTTGCCAAC | 44 |
| 2_P_{FBA1}_F | CGATGCCATATTGTATGTGTATTGT | 45 |
| 2_P_{FBA1}_R | TGTGTTTGTGTTGGGGGTGGTAG | 46 |
| 3_SbMDH_F | ACTACCACCCCCAACACAAACACAATGGGTTTGTCT | 47 |
| | ACCGCATACTCTC | |
| 3_SbMDH_R | CCCTTGAACAAACATCTTGTTGTAGTTAAACTTCAC CTGGCAACATAGT | 48 |
| 4_T_{INO1}_F | CTACAACAAGATGTTTGTTCAAGG | 49 |
| 4_T_{lNO1}_R | GAAGATTACCATACCATCCCACC | 50 |
| 5_DW_2365_F1 | GATTGGTGGGATGGTATGGTAATCTTCGATGAGCAT CTAGAGTTGAAGAGGG | 51 |
| 5_DW_2365_F2 | GTTGGCAAGATTCGATAGTATTCAGGATGAGCATCT AGAGTTGAAGAGGG | 52 |
| 5_DW_2365_R | CCGACTTGTACGAATTTGTTCCTC | 53 |
| 2365_sgRNA-1R | AACTTGCTATTTCTAGCTCTAAAACCTCTCGGATCA TCCTTTGGCTGCGCAAGCCCGGAATCGAA | 54 |

### 3. Construction of a strain over-expressing SpMAE1 and SbMDH genes

The pWSPK_MCH4 plasmid and Fragments 6 and 7 were electrically transferred into a CY902 *ΔURA3* strain according to a yeast electrotransformation method (doi:10.1111/1751-7915.13781), and a positive transformant was screened and obtained, which was named MA101-1 (genotype: CY902 *ΔURA3*, *ΔPkMCH4*); the pWSPK_MCH4 plasmid and Fragments 1-5 were electrically transferred into a CY902 *ΔURA3* strain and screened to obtain a positive transformant which was named MA101-2 (genotype: CY902 *ΔURA3, PkMCH4::P_{PkTDH3}-ORF_{SpMAE1}-T_{PkGAL2}*).

The pWSPK_2365 plasmid and Fragments 13 and 14 were transferred into a MA101-2 strain according to a yeast electrotransformation method, and a positive transformant was screened and obtained, which was named MA102-1 strain (genotype: MA101-2, *ΔPk2365*); the pWSPK_2365 plasmid and Fragments 8-12 were transferred into a MA101-2 strain according to the yeast electrotransformation method, and a positive transformant was screened and obtained, which was named MA102-2 strain (genotype: MA101-2, *Pk2365::P_{PkFBA1}-ORF_{SbMDH}-T_{PkINO1}*).

### Example 2: Evaluation of L-malate production capacity of MA101 and MA102 series

### 1. Fermentation with a neutralizing agent

A 30 mL yeast inorganic salt culture medium (5% w/v glucose, 30 g/L CaCOs) was inoculated respectively with MA101-1, MA101-2, MA102-1 and MA102-2 strains to undergo flask shaking fermentation for 30 h at 30°C at 250 rpm, and the titer of L-malate was measured by HPLC, as shown in Table 2.

**Table 2. Flask shaking fermentation titer of L-malate of MA101 and MA102 series strains**

| strain | MA101-1 | MA101-2 | MA 102-1 | MA 102-2 |
|---|---|---|---|---|
| Malate, g/L | 0 | 13.22 | 15.04 | 23.29 |

### 2. Fermentation without a neutralizing agent

A 30 mL yeast inorganic salt culture medium (5% w/v glucose, 0g/L CaCOs) was inoculated respectively with MA101-1, MA101-2, MA102-1 and MA102-2 strains to undergo flask shaking fermentation for 30 h at 30°C at 250 rpm, and the titer of L-malate was measured by HPLC, as shown in Table 3.

**Table 3 Flask shaking fermentation titer of L-malate of MA101 and MA102 series strains**

| strain | MA101-1 | MA101-2 | MA 102-1 | MA 102-2 |
|---|---|---|---|---|
| Malate, g/L | 0 | 0 | 0 | 2.14 |

In view of the fact that the highest L-malate titer was obtained from the MA102-2 strain, it was used for construction of subsequent L-malate strains.

### Example 3: knockout of PDC1 and GPD1 genes in MA102-2 strain

### 1. Construction of DNA fragments for knocking out PDC1 and GPD1

Using CY902 genome DNA as a template, upstream and downstream homologous arm fragments (abbreviated as PDC1_1 and PDC1_2) of *PDC1* gene were amplified with primers PDC1_1F and PDC1_1R as well as PDC1_2F and PDC1_2R (see Table 4); upstream and downstream homologous arm fragments (abbreviated as GPD1_1 and GPD1_2) of *GPD1* gene were amplified with primers GPD1_1F and GPD1_1R as well as GPD1_2F and GPD1_2R (see Table 4).

### 2. Construction of CRISPR/Cas9 plasmids for editing PDC1 and GPD1 genes

The 5'-end sequence (abbreviated as PDC1_sgRNA_1, SEQ ID NO: 102) of sgRNA was amplified with primers sgRNA-1F and PDC1_sgRNA-1R (see Table 4), and the 3' terminal of this fragment contained a 20 nt protospacer specific to *PDC1* gene. The plasmid backbone pWSPK_backbone and sgRNA fragments PDC1_sgRNA_1 and sgRNA_2 were ligated by using a seamless cloning kit. The seamless cloneing ligated product was transferred into Trans1-T1 competent cells, and the obtained positive plasmid was named pWSPK_PDC1. The 5'-end sequence (abbreviated as GPD1_sgRNA_1) of sgRNA was amplified by with primers sgRNA-1F and GPD1_sgRNA-1R (see Table 4), and the 3' terminal of this fragment contained a 20 nt protospacer specific to *GPD1* gene. The pWSPK _backbone, sgRNA_2 and GPD1_sgRNA_1 were fused by seamless cloning to form a pWSPK_GPD1 plasmid for editing the *GPD1* gene.

**Table 4. Primers required for knockout of PDC1 and GPD1 genes and construction of CRISPR/Cas9 plasmids**

| Primer Name | Sequence (5'-3'), protospacer underlined | SEQ ID NO |
|---|---|---|
| PDC1_1F | CCGTATCTTATTGTATCCTATCCTATTC | 55 |
| PDC1_1R | ATGTTCATTTTACATTCAGATGTCAATTTTTATGTTTTGC TGTGTTTTGTTTTA | 56 |
| PDC1_2F | ACATAAAAATTGACATCTGAATGTAAAATGAACATTAAA | 57 |
| PDC1_2R | GTTGAAGTCAACGAAGATGGTGAG | 58 |
| PDC1_sgRNA-1R | AACTTGCTATTTCTAGCTCTAAAACGCATTCAACTCGT TAGCGTTTGCGCAAGCCCGGAATCGAA | 59 |
| GPD1_1F | CTCTACAAGAGAGGAAACACACTACAG | 60 |
| GPD1_1R | AAAATAAATTTAAAATAAACGATATCAAAATTCA | 61 |
| GPD1_2F | GAATTTTGATATCGTTTATTTTAAATTTATTTTATAACC TCAAGGAGAACTTTGGCATT | 62 |
| GPD1_2R | GAAATCTATGCAAACCACTCTCTCCTG | 63 |
| GPD1_sgRNA-1R | AACTTGCTATTTCTAGCTCTAAAACGCAGATTCATGA GTAAAGGTTGCGCAAGCCCGGAATCGAA | 64 |

### 3. Construction of a strain with PDC1 and GPD1 genes knocked out

The fragments PDC1_1 and PDC1_2 and plasmid pWSPK_PDC1 were simultaneously transferred into MA102-2. The positive transformant was identified with primers PDC1_1F/PDC1_2R, and was named MA103 strain (genotype: MA102-2, *ΔPDC1*). The pWSPK *GPD1* plasmid and fragments GPD1_1 and GPD1_2 were electrically transferred into a MA103 strain, and a positive transformant was screened and obtained via SD-URA culture medium, which was named a MA104 strain (genotype: MA103, *ΔGPD1*).

### Example 4: overexpression of carboxylase genes in MA102-2 strain

Three carboxylases, including (I) *Escherichia coli* phosphoenolpyruvate carboxykinase EcPCK (Uniprot database search number: P22259); (II) *Escherichia coli* phosphoenolpyruvate carboxylase EcPPC (Uniprot database search number: P00864), were over-expressed at the *JEN*2-2 site, respectively. PkPCK1 (loci number PK0402, SEQ ID NO: 6) of CY902 itself was used as control. The promoter and terminator used were respectively a promoter of enolase gene *ENO1* and a terminator of GPI-cell wall glycoprotein gene *SED1* of CY902 itself. *Aspergillus oryzae*-derived AoPYC (Uniprot database search number: Q2UGL1) (SEQ ID NO: 4) was respectively over-expressed at *JEN2-1* site, PkPYC (SEQ ID NO: 5) of CY902 itself was used as control. The promoter and terminator used were respectively a *TDH3* promoter and a *GAL2* terminator. *EcPCK, EcPPC* and *AoPYC* genes were synthesized by Nanjing GenScript Biotech Co., Ltd, and optimized according to CY902 codon preference. The specific construction method was as follows:

### 1. Construction of donor DNA fragments for homologous recombination

Using CY902 genome DNA as a template, an upstream homologous arm fragment 1 (Fragment 15) of *JEN*2-2 gene was amplified with primers 1_UP_JEN2-2_F and 1_UP_JEN2-2_R1 (see Table 5); a promoter sequence (Fragment 16) of *ENO1* gene was amplified with primers 2_P_{ENO1}_F and 2_P_{ENO1}_R (see Table 5); using a plasmid containing *EcPCK* synthetic sequence as a template, an EcPCK encoding sequence (Fragments 17-1, SEQ ID NO: 7) of *Escherichia coli* was amplified with primers 3_EcPCK_F and 3_EcPCK_R (see Table 5); using a plasmid containing *EcPPC* synthetic sequence as a template, an EcPPC encoding sequence (Fragments 17-2, SEQ ID NO: 8) of *Escherichia coli* was amplified with primers 3_EcPPC_F and 3_EcPPC_R (see Table 5); using CY902 genome DNA as a template, a PkPCK encoding sequence (Fragment 17-3) was amplified with primers 3_PkPCK_F and 3_PkPCK_R (see Table 5); a terminator sequence (Fragment 18) of *SED1* gene was amplified with primers 4_T_{SED1}_F and 4_T_{SED1}_R (see Table 5); an downstream homologous arm fragment 1 (Fragment 19) of *JEN2-2* gene was amplified with primers 5_DW_JEN2-2_F1 and 5_DW_JEN2-2_R (see Table 5); an upstream homologous arm fragment 2 (Fragment 20) of *JEN2-2* gene was amplified with primers 1_UP_JEN2-2_F and 1_UP_JEN2-2_R2 (see Table 5); a downstream homologous arm fragment 2 (Fragment 21) of *JEN2*-*2* gene was amplified with primers 5_DW_JEN2-2_F2 and 5_DW_JEN2-2_R (see Table 5).

Using CY902 genome DNA as a template, an upstream homologous arm fragment 1 (Fragment 22) of *JEN2-1* gene was amplified with primers 1_UP_JEN2-1_F and 1_UP_JEN2-1_R1 (see Table 5); a PkPYC1 encoding sequence (Fragment 23-1) of CY902 itself was amplified with primers 3_PkPYC1_F and 3_PkPYC1_R (see Table 5); using a plasmid containing *AoPYC* synthetic sequence as a template, an *Aspergillus oryzae*-derived AoPYC encoding sequence (Fragment 23-2, SEQ ID NO: 4) was amplified with primers 3_AoPYC_F and 3_AoPYC_R (see Table 5); using CY902 genome DNA as a template, a downstream homologous arm fragment 1 (Fragment 24) of *JEN2-1* gene was amplified with primers 5_DW_JEN2-1_F1 and 5_DW_JEN2-1_R (see Table 5). Using CY902 genome DNA as a template, an upstream homologous arm fragment 2 (Fragment 25) of *JEN2-1* gene was amplified with primers 1_UP_JEN2-1_F and 1_UP_JEN2-1_R2 (see Table 5); a downstream homologous arm fragment 2 (Fragment 26) of *JEN2-1* gene was amplified with primers 5_DW_JEN2-1_F2 and 5_DW_JEN2-1_R (see Table 5).

**Table 5. Primers required for overexpression of carboxylase genes and construction of CRISPR/Cas9 plasmids**

| Primer Name | Sequence (5'-3'), protospacer underlined | SEQ ID NO |
|---|---|---|
| 1_UP_JEN2-2_F | CCTTGGACCAACCAATAACCTGATTC | 65 |
| 1_UP_JEN2-2_R1 | GGTGTCTTTGGCGAAGTGTTGTTGTTGCTATCTTTTGGT | 66 |
| | ATTGTGCATAATCA | |
| 1_UP_JEN2-2_R2 | TTGCTATCTTTTGGTATTGTGCATAATCA | 67 |
| 2_P_{ENO1}_F | CAACAACACTTCGCCAAAGACAC | 68 |
| 2_P_{ENO1}_R | TGTTTGGTTGGAGGGGGTTATATG | 69 |
| 3_EcPCK_F | CATATAACCCCCTCCAACCAAACAATGAGAGTTAACAA CGGTTTGAC | 70 |
| 3_EcPCK_R | CAAAAGAAAGAGCAGTAACCTAATCCACTTATAACTTT GGACCTGCAGCAAC | 71 |
| 3_EcPPC_F | CATATAACCCCCTCCAACCAAACAATGAACGAACAATA CTCTGCTTTG | 72 |
| 3_EcPPC_R | CAAAAGAAAGAGCAGTAACCTAATCCACTTAACCAGT GTTTCTCATACCAG | 73 |
| 3_PkPCK_F | CATATAACCCCCTCCAACCAAACAATGGCTCCAACTGC TGTTGATATC | 74 |
| 3_PkPCK_R | CAAAAGAAAGAGCAGTAACCTAATCCACTTATAAAGCA GGACCAGCAGCAAC | 75 |
| 4_T_{SED1}_F | GTGGATTAGGTTACTGCTCTTTCTTTTG | 76 |
| 4_T_{SED1}_R | CTTGGTTGGATTTGGTATAGTTGAG | 77 |
| 5_DW_JEN2-2_F1 | CTCTCAACTATACCAAATCCAACCAAGCAGAAGCTTCC ATGTATGATGTATG | 78 |
| 5_DW_JEN2-2_F2 | TATGCACAATACCAAAAGATAGCAACAGCAATGTTAAC CCCAGAAGCT | 79 |
| 5_DW_JEN2-2_R | GCAGTTTCTGCAAGATTTCACACAG | 80 |
| JEN2-2_sgRNA-1R | AACTTGCTATTTCTAGCTCTAAAACGGCAATGGATTCA ACAAATGTGCGCAAGCCCGGAATCGAA | 81 |
| 1_UP_JEN2-1_F | GATAGGTTCAAAGCCCTCGTCG | 82 |
| 1_UP_JEN2-1_R1 | GAAGTGATGTAGATACGCCAACAGGGGTTGTGTTTCAA GCACCTGACT | 83 |
| 1_UP_JEN2-1_R2 | GGTTGTGTTTCAAGCACCTGACT | 84 |
| 3_PkPYC1_F | CAAAACACACAAACAAACACAATTACAAAAAATGTCA ACTGTGGAAGATCACTCCTC | 85 |
| 3_PkPYC1_R | ATTAAGACAATTGACAGAAAGTGATTTAAGCTGCTGGC GCTTCATCTTTG | 86 |
| 3_AoPYC_F | CAAAACACACAAACAAACACAATTACAAAAAATGGCT GCACCATTCAGACAAC | 87 |
| 3_AoPYC_R | ATTAAGACAATTGACAGAAAGTGATTTAAGCCTTAACA ATCTTACAAACC | 88 |
| 5_DW_JEN2-1_F1 | GAATACTAGCTAAATCGGTATCGCCCACACCCCTGTGG ACCTGTTCTAT | 89 |
| 5_DW_JEN2-1_F2 | AGTCAGGTGCTTGAAACACAACCCCCTGTGGACCTGTT CTATGTAAAC | 90 |
| 5_DW_JEN2-1_R | ATTCCTCCCATTTCTACCCCCTG | 91 |
| JEN2-1_sgRNA-1R | AACTTGCTATTTCTAGCTCTAAAACTCAAGTGTTCATT ATTGTGATGCGCAAGCCCGGAATCGAA | 92 |

### 2. Construction of CRISPR/Cas9 plasmids for editing JEN2-1 and JEN2-2 genes

Using a pWSPK-Cas9 plasmid as a template, the 5'-end sequence (abbreviated as JEN2-1_sgRNA_1) of sgRNA was amplified with primers sgRNA-1F and JEN2-1_sgRNA-1R (see Table 5), and the 3' terminal of this fragment contained a 20 nt protospacer specific to *JEN2-1* gene. The pWSPK _backbone, sgRNA _2 and JEN2-1_sgRNA_1 in Example 1 were ligated by using a seamless cloning kit to finally obtain a positive clone plasmid which was named pWSPK_JEN2-1.

Using a pWSPK-Cas9 plasmid as a template, the 5'-end sequence (abbreviated as JEN2-2_sgRNA_1) of sgRNA was amplified with primers sgRNA-1F and JEN2-2_sgRNA-1R (see Table 5), and the 3' terminal of this fragment contained a 20 nt protospacer specific to *JEN2-2* gene. The pWSPK_backbone, sgRNA_2 and JEN2-2_sgRNA_1 were ligated by using a seamless cloning kit to finally obtain a positive clone plasmid which was named pWSPK _JEN2-2.

### 3. Construction of strains over-expressing carboxylase genes

The pWSPK_JEN2-2 plasmid and Fragments 20 and 21 were electrically transferred into a MA102-2 strain, and a positive transformant was screened and obtained, which was named MA105-1 (genotype: MA102-2, *ΔPkJEN2-2*); the pWSPK_JEN2-2 plasmid and Fragments 15-19 were electrically transferred into MA102-2 strain, and positive transformants was screened and obtained, which were respectively named MA105-2 (genotype: MA102-2, *JEN2-2::P_{PkENO1}-ORF_{EcPCK}-T_{PksED1}*); MA105-3 (genotype: MA102-2, *JEN2-2::P_{PkENO1}-ORF_{EcPPC}-T_{PkSED1}*); and MA105-4 (genotype: MA102-2, *JEN2-2::P_{PkENO1}-ORF_{PkPCK}-T_{PkSED1}*); the pWSPK_JEN2-1 plasmid and Fragments 25 and 26 were electrically transformed into a MA102-2 strain, and a positive transformant was screened and obtained, which was named MA105-5 (genotype: MA102-2, *ΔPkJEN2-1*); the pWSPK *JEN2-1* plasmid and Fragments 22, 2, 23, 4 and 24 were electrically transferred into MA102-2 strain, and positive transformants were screened and obtained, which were named MA105-6 (genotype: MA102-2, *PkJEN2-1::P_{PkTDH3}-ORF_{PkPYC1}-T_{PkGAL2}*); MA105-7 (genotype: MA102-2, *PkJEN2-1::P_{PkTDH3}-ORF_{AoPYC}-T_{PkGAL2}*).

### Example 5: Overexpression of SpVHT1 gene

*Schizosaccharomyces pombe-derived* biotin transport protein SpVHT1 (Uniprot database search number: 013880) was over-expressed at the *PkMAE1* site of the MA102-2 strain. The *SpVHT1* gene sequence (SEQ ID NO: 3) was optimized according to CY902 codon preference by Nanjing GenScript Biotech Co., Ltd.

The promoter and terminator used were respectively a *TDH3* promoter and a *GAL2* terminator.

### 1. Construction of donor DNA fragments for homologous recombination

Using CY902 genome DNA as a template, an upstream homologous arm fragment 1 (Fragment 27) of *PkMAE1* gene was amplified with primers 1_UP_MAE1_F and 1_UP_MAE1_R1 (see Table 6); using a plasmid containing *SpVHT1* synthetic sequence as a template, a SpVHT1 encoding sequence (Fragment 28) of *Schizosaccharomycespombe* was amplified with primers 3_SpVHT1_F and 3_SpVHT1_R (see Table 6); using CY902 genome DNA as a template, a downstream homologous arm fragment 1 (Fragment 29) of a *PkMAE1* gene was amplified with primers 5_DW_MAE1_F1 and 5_DW_ MAE1_R (see Table 6). Using CY902 genome DNA as a template, an upstream homologous arm fragment 2 (Fragment 30) of a *PkMAE1* gene was amplified with primers 1_UP_MAE1_F and 1_UP_MAE1_R2 (see Table 6); a downstream homologous arm fragment 2 (Fragment 31) of *PkMAE1* gene was amplified with primers 5_DW_MAE1_F2 and 5_DW_MAE1_R (see Table 6).

### 2. Construction of CRISPR/Cas9 plasmid for editing PkMAE1 gene

Using a pWSPK-Cas9 plasmid as a template, the 5'-end sequence (abbreviated as MAE1_sgRNA_1) of sgRNA was amplified with primers sgRNA-1F and MAE1_sgRNA-1R (see Table 6), and the 3' terminal of this fragment contained a 20 nt protospacer specific to *PkMAE1* gene. The pWSPK_backbone, sgRNA_2 and MAE1_sgRNA_1 were ligated by using a seamless cloning kit to finally obtain a positive clone plasmid which was named pWSPK_MAE1.

**Table 6. Primers for construction of SpVHT1 gene over-expressing fragments and of a CRISPR/Cas9 plasmid**

| Primer Name | Sequence (5'-3'), protospacer underlined | SEQ ID NO |
|---|---|---|
| 1_UP_MAE1_F | TGGAAGACGCACTAGTCTCAAAGG | 93 |
| 1_UP_MAE1_R1 | GAAGTGATGTAGATACGCCAACAGGGGCGTGTT CTTTTTAATTATTGCTCA | 94 |
| 1_UP_MAE1_R2 | GGCGTGTTCTTTTTAATTATTGCTCA | 95 |
| 3_SpVHT1_F | CAAAACACACAAACAAACACAATTACAAAAAA TGGCATCCGAATGGCCAGAAAC | 96 |
| 3_SpVHT1_R | ATTAAGACAATTGACAGAAAGTGATTTATGCGT CTGCTTGTTGGGAGGAT | 97 |
| 5_DW_MAE1_F1 | GAATACTAGCTAAATCGGTATCGCTGGACACATA CACATTATCAAATGCA | 98 |
| 5_DW_MAE1_F2 | GAGCAATAATTAAAAAGAACACGCCTGGACACA TACACATTATCAAATGC | 99 |
| 5_DW_MAE1_R | GCTAAGCACACCGGCCTTGGAAAC | 100 |
| MAE1_sgRNA-1R | AACTTGCTATTTCTAGCTCTAAAACCCTCTCCCA CTGGAATCTTTTGCGCAAGCCCGGAATCGAA | 101 |

### 3. Construction of a strain over-expressing SpVHT1 gene

The pWSPK_MAE1 plasmid and Fragments 30 and 31 were electrically transferred into a MA102-2 strain according to yeast electrotransformation method, and a positive transformant was screened and obtained, which was named MA106-1 strain (genotype: MA102-2, *ΔPkMAE1*); the pWSPK_MAE1 plasmid and Fragments 27, 2, 28, 4 and 29 were transferred into a MA102-2 strain according to the yeast electrotransformation method, and a positive transformant was screened to obtained, which was named MA106-2 strain (genotype: MA102-2, *PkMAE1::P_{PkTDH3}-ORF_{SpVHT1}-T_{PkGAL2}*).

### Example 6: Complementary expression of PkURA3 gene in MA101 and MA102 series strains

A orotidine 5'-phosphate decarboxylase gene *PkURA3* (SEQ ID NO: 104) was complementary at the *PkMCHS* locus of monocarboxylate permease (NCBI Reference Sequence: XP_029319985.1) of MA101 and MA102 series strains (NCBI Reference Sequence: XP_029319985.1). The promoter and terminator used were a promoter (SEQ ID NO: 105) and a terminator (SEQ ID NO: 106) of *PkURA3* itself. The specific construction method was as follows:

### 1. Construction of donor DNA fragments for homologous recombination

Using CY902 genome DNA as a template, an upstream homologous arm fragment 1 (Fragment 32) of *PkMCH5* gene of CY902 itself was amplified with primers 1_UP_MCH5_F and 1_UP_MCH5_R1 (see Table 7); a *URA3* promoter, an encoding frame and a terminator sequence (Fragment 33) were amplified with primers 2_PkURA3_F and 2_PkURA3_R (see Table 7); a downstream homologous arm fragment 1 (Fragment 34) of *PkMCH5* gene of CY902 itself was amplified with primers 3_DW_MCH5_F1 and 3_DW_MCH5_R (see Table 7). Using CY902 genome DNA as a template, an upstream homologous arm fragment 2 (Fragment 35) of *PkMCH5* gene of CY902 itself was amplified with primers 1_UP_MCH5_F and 1_UP_MCH5_R2 (see Table 7); a downstream homologous arm fragment 2 (Fragment 36) of *PkMCH5* gene of CY902 itself was amplified with primers 3_DW_MCH5_F2 and 3_DW_MCH5_R (see Table 7).

### 2. Construction of a plasmid for editing PkMCHS locus

Using a pWSPK-Cas9 plasmid as a template, the 5'-end sequence (abbreviated as MCH5_sgRNA_1) of sgRNA was amplified with primers sgRNA-1F and MCH5_sgRNA-1R (see Table 7), and the 3' terminal of this fragment contained a 20 nt protospacer specific to *PkMCHS* gene promoter. The pWSPK_backbone, sgRNA_2 and MCH5_sgRNA_1 were ligated by using a seamless cloning kit to finally obtain a positive clone plasmid which was named pWSPK _MCH5.

**Table 7. Primers required for complementary expression of PkURA3 gene and construction of a CRISPR/Cas9 plasmid**

| Primer Name | Sequence (5'-3'), protospacer underlined | SEQ ID NO |
|---|---|---|
| 1_UP_MCH5_F | GTGCAGGAATTTCAAGATAGTCGG | 107 |
| 1_UP_MCH5_R1 | CTTCCAATATAACTGACGGACTATACCCCTTGTG CTCTATCTCTATGTTCCC | 108 |
| 1_UP_MCH5_R2 | CCTTGTGCTCTATCTCTATGTTCCC | 109 |
| 2_PkURA3_F | GGTATAGTCCGTCAGTTATATTGGAAGA | 110 |
| 2_PkURA3_R | GAAGACGTTCATGTATGTTTCTGTTG | 111 |
| 3_DW_MCH5_F1 | AACAGAAACATACATGAACGTCTTCTTACATCC GAGTCTGTTGTATTGCC | 112 |
| 3_DW_MCH5_F2 | GGGAACATAGAGATAGAGCACAAGGTTACATCC GAGTCTGTTGTATTGCC | 113 |
| 3_DW_MCH5_R | ATGGGGTCTCCTGAACATTTGTG | 114 |
| MCH5_sgRNA-1R | AACTTGCTATTTCTAGCTCTAAAACCGATCGCA CGAACTACTGTTTGCGCAAGCCCGGAATCGAA | 115 |

### 3. Construction of strains with complementary PkURA3

The pWSPK _MCH5 plasmid and Fragments 35 and 36 were transferred into MA101-1, MA101-2, MA102-1 and MA102-2 strains according to the yeast electrotransformation method, and positive transformants were screened and obtained, which were named MA107-1 strain (genotype: MA101-1, *ΔPkMCH5*), MA107-2 strain (genotype: MA101-2, *ΔPkMCH5*), MA108-1 strain (genotype: MA102-1, *ΔPkMCH5*) and MA108-2 strain (genotype: MA102-2, *ΔPkMCH5*), respectively. The pWSPK_MCH5 plasmid and Fragments 32-34 were transferred into MA101-1, MA101-2, MA102-1 and MA102-2 strains according to the yeast electrotransformation method, and positive transformants were screened and obtained, which were named MA107-3 strain (genotype: MA101-1, *PkMCH5::P_{PkURA3}-ORF_{PkURA3}-T_{PkURA3}*), MA107-4 strain (genotype: MA101-2, *PkMCH5::P_{PkURA3}-ORF_{PkURA3}-T_{PkURA3}*), MA108-3 strain (genotype: MA102-1, *PkMCH5::P_{PkURA3}-ORF_{PkURA3}-T_{PkURA3})* and MA108-4 strain (genotype: MA102-2, *PkMCH5::P_{PkURA3}-ORF_{PkURA3}-T_{PkURA3}*), respectively.

### Example 7: Complementary expression of PkURA3 gene in MA103-MA106 series strains

With reference to Example 6, *PkURA3* was complementary at the *PkMCHS* locus of MA103-MA106 series strains to obtain MA109 strain (genotype: MA103, *PkMCH5::P_{PkURA3}-ORF_{PkURA3}-T_{PkURA3}*); MA110 strain (genotype: MA104, *PkMCH5::P_{PkURA3}-ORF_{PkURA3}-T_{PkURA3}*); MA111-1 strain (genotype: MA105-1, *PkMCH5::P_{PkURA3}-ORF_{PkURA3}-T_{PkURA3}*); MA111-2 strain (genotype: MA105-2, *PkMCH5::P_{PkURA3}-ORF_{PkURA3}-T_{PkURA3}*); MA111-3 strain (genotype: MA105-3, *PkMCH5::P_{PkURA3}-ORF_{PkURA3}-T_{PkURA3}*); MA111-4 strain (genotype: MA105-4, *PkMCH5::P_{PkURA3}-ORF_{PkURA3}-T_{PkURA3}*); MA111-5 strain (genotype: MA105-5, *PkMCH5::P_{PkURA3}-ORF_{PkURA3}-T_{PkURA3}*); MA111-6 strain (genotype: MA105-6, *PkMCH5::P_{PkURA3}-ORF_{PkURA3}-T_{PkURA3}*); MA111-7 strain (genotype: MA105-7, *PkMCH5::P_{PkURA3}-ORF_{PkURA3}-T_{PkURA3}*); MA112-1 strain (genotype: MA106-1, *PkMCH5::P_{PkURA3}-ORF_{PkURA3}-T_{PkURA3}*); MA112-2 strain (genotype: MA106-2, *PkMCH5::P_{PkURA3}-ORF_{PkURA3}-T_{PkURA3}*)*.*

### Example 8: Evaluation of L-malate production capacity of MA101-MA112 series strains

### 1. Fermentation with a neutralizing agent

A 30 mL yeast inorganic salt culture medium (5% w/v glucose, 30 g/L CaCOs) was inoculated with MA101-MA112 series strains respectively, and then subjected to shaking fermentation for 30 h at 30°C under 250 rpm, and the titer of L-malate was measured by HPLC.

**Table 8. Flask shaking fermentation titer of malate of MA101-MA112 series strains**

| Strain | MA101-1 | MA101-2 | MA102-1 | MA102-2 | MA103 | MA104 |
|---|---|---|---|---|---|---|
| Malate, g/L | 0 | 13.22 | 15.04 | 23.29 | 23.57 | 26.43 |

| Strain | MA105-1 | MA105-2 | MA105-3 | MA105-4 | MA105-5 | MA105-6 |
|---|---|---|---|---|---|---|
| Malate, g/L | 23.53 | 26.15 | 27.35 | 26.42 | 23.94 | 25.66 |

| Strain | MA105-7 | MA106-1 | MA 106-2 | MA107-1 | MA107-2 | MA 107-3 |
|---|---|---|---|---|---|---|
| Malate, g/L | 26.24 | 24.27 | 25.11 | 0 | 13.41 | 0 |

| Strain | MA107-4 | MA108-1 | MA108-2 | MA108-3 | MA108-4 | MA109 |
|---|---|---|---|---|---|---|
| Malate, g/L | 13.58 | 15.17 | 22.95 | 15.33 | 23.66 | 25.11 |

| Strain | MA110 | MA111-1 | MA111-2 | MA111-3 | MA111-4 | MA111-5 |
|---|---|---|---|---|---|---|
| Malate, g/L | 27.45 | 23.94 | 26.31 | 27.94 | 26.79 | 24.77 |

| Strain | MA111-6 | MA111-7 | MA112-1 | MA112-2 | | |
|---|---|---|---|---|---|---|
| Malate, g/L | 26.20 | 26.97 | 24.71 | 25.43 | | |

### 2. Fermentation without a neutralizing agent

A 30 mL yeast inorganic salt culture medium (5% w/v glucose, 0 g/L CaCOs) was inoculated with MA101-MA112 series strains respectively, and then subjected to shaking fermentation for 30 h at 30°C under 250 rpm, and the titer of L-malate was measured by HPLC.

**Table 9. Flaks shaking fermentation titer of malate of MA101-MA112 series strains**

| Strain | MA101-1 | MA101-2 | MA102-1 | MA102-2 | MA103 | MA104 |
|---|---|---|---|---|---|---|
| Malate, g/L | 0 | 0 | 0 | 2.14 | 3.93 | 4.84 |

| Strain | MA105-1 | MA105-2 | MA105-3 | MA105-4 | MA105-5 | MA105-6 |
|---|---|---|---|---|---|---|
| Malate, g/L | 2.31 | 3.74 | 4.22 | 3.92 | 2.44 | 3.24 |

| Strain | MA 105-7 | MA 106-1 | MA 106-2 | MA107-1 | MA107-2 | MA107-3 |
|---|---|---|---|---|---|---|
| Malate, g/L | 3.63 | 2.47 | 2.96 | 0 | 0 | 0 |

| Strain | MA107-4 | MA108-1 | MA108-2 | MA108-3 | MA108-4 | MA 109 |
|---|---|---|---|---|---|---|
| Malate, g/L | 0 | 0 | 2.11 | 0 | 2.38 | 4.14 |

| Strain | MA110 | MA111-1 | MA111-2 | MA111-3 | MA111-4 | MA111-5 |
|---|---|---|---|---|---|---|
| Malate, g/L | 4.91 | 2.36 | 3.92 | 4.76 | 4.18 | 2.57 |

| Strain | MA111-6 | MA111-7 | MA112-1 | MA112-2 | | |
|---|---|---|---|---|---|---|
| Malate, g/L | 3.51 | 3.79 | 2.62 | 3.01 | | |

### Example 9: Optimization of L-malate-producing strain

With reference to Example 4, three carboxylase genes were over-expressed in MA104 to obtain MA113-1 strain (genotype: MA104, *ΔPkJEN2-2*); MA113-2 strain (genotype: MA104, *JEN2-2::P_{PkENO1}-ORF_{EcPCK}-T_{PkSED1}*); MA113-3 strain (genotype: MA104, *JEN2-2::P_{PkENO1}-ORF_{EcPPC}-T_{PkSED1}*); MA113-4 strain (genotype: MA104, *JEN2-2::P_{PkENO1}-ORF_{PkPCK}-T_{PkSED1}*). *PYCs* from two origins were over-expressed in the MA113-3 strain to obtain MA114-1 strain (genotype: MA113-3, *ΔPkJEN2-1*); to obtain MA114-2 strain (genotype: MA113-3, *PkJEN2-1::P_{PkTDH3}-ORF_{PkPYC1}-T_{PkGAL2}*); MA114-3 strain (genotype: MA113-3, *PkJEN2-1::P_{PkTDH3}-ORF_{AoPYC}-T_{PkGAL2}*).

With reference to Example 5, biotin transport protein SpVHT1 was over-expressed in MA1 14-3 to obtain MA115-1 strain (genotype: MA114-3, *ΔPkMAE1*); MA115-2 strain (genotype: MA114-3, *PkMAE1::P_{PkTDH3}-ORF_{SpVHT1}-T_{PkGAL2}*).

With reference to Example 6, orotidine 5'-phosphate decarboxylase gene *PkURA3* was complementary in the MA115-2 strain to obtain MA116 strain (genotype: MA115-2, *PkMCH5::P_{PkURA3}-ORF_{PkURA3}-T_{PkURA3}*).

### Example 10: Evaluation of L-malate production capacity of MA116 strain

MA116 strain was cultured in a 5 L tank (a yeast inorganic culture medium, 20% w/v glucose, 70 g/L CaCOs) for fermentation, without control of fermentation pH. The 120 h titer of malate reached 204 g/L.

### Example 11: overexpression of SpMAEl and SbMDH genes in Pichia kudriavzevii CICC32244 ΔURA3 strain

With reference to Example 1, a CICC32244 (purchased from China Center of Industrial Culture Collection) orotidine 5'-phosphate decarboxylase encoding gene *URA3* was knocked out through homologous recombination to obtain a CICC32244 Δ*URA3* mutant.

The pWSPK_MCH4 plasmid and Fragments 6 and 7 were electrically transferred into a CICC32244 *ΔURA3* strain according to a yeast electrotransformation method, and a positive transformant was screened and obtained, which was named MA117-1 (genotype: CICC32244 *ΔURA3, ΔPkMCH4*); the pWSPK_MCH4 plasmid and Fragments 1-5 were electrically transferred into a CICC32244 *ΔURA3* strain and a positive transformant was screened and obtained, which was named MA117-2 (genotype: CICC32244 *ΔURA3, PkMCH4::P_{PkTDH3}-ORF_{SpMAE1}-T_{PkGAL2}*).

The pWSPK_2365 plasmid and Fragments 13 and 14 were electrically transferred into a MA117-2 strain according to the yeast electrotransformation method, and a positive transformant was screened and obtained, which was named MA118-1 (genotype: MA117-2, *ΔPk2365*); the pWSPK_2365 plasmid and Fragments 8-12 were electrically transferred into a MA117-2 strain according to the yeast electrotransformation method, and a positive transformant was screened and obtained, which was named MA118-2 strain (genotype: MA117-2, *Pk2365::P_{PkFBA1}-ORF_{SbMDH}-T_{PkINO1}*).

### Example 12: Complementary expression of PkURA3 gene in MA117 and MA118 series strains

With reference to Example 6, *PkURA3* was complementary at *PkMCHS* locus of MA117 and MA118 series strains to obtain MA119-1 strain (genotype: MA117-1, *PkMCH5::P_{PkURA3}-ORF_{PkURA3}-T_{PkURA3}*); MA119-2 strain (genotype: MA117-2, *PkMCH5::P_{PkURA3}-ORF_{PkURA3}-T_{PkURA3}*); MA120-1 strain (genotype: MA118-1, *PkMCH5::P_{PkURA3}-ORF_{PkURA3}-T_{PkURA3}*); MA120-2 strain (genotype: MA118-2, *PkMCH5::P_{PkURA3}-ORF_{PkURA3}-T_{PkURA3}*).

### Example 13: Evaluation of malate production capacity of MA117-MA120 series strains

### 1. Fermentation with a neutralizing agent

A 30 mL yeast inorganic salt culture medium (5% w/v glucose, 30 g/L CaCOs) was inoculated with MA117-MA120 series strains respectively, and subjected to shaking fermentation for 30 h at 30°C under 250 rpm, and the titer of L-malate was measured by HPLC.

**Table 10. Flask shaking fermentation titer of malate of MA117-MA120 series strains**

| Strain | MA117-1 | MA117-2 | MA118-1 | MA118-2 |
|---|---|---|---|---|
| Malate, g/L | 0 | 6.20 | 6.82 | 10.75 |

| Strain | MA119-1 | MA119-2 | MA120-1 | MA120-2 |
|---|---|---|---|---|
| Malate, g/L | 0 | 6.73 | 7.11 | 11.43 |

### 2. Fermentation without a neutralizing agent

A 30 mL yeast inorganic salt culture medium (5% w/v glucose, 0 g/L CaCOs) was inoculated with MA117-MA120 series strains respectively, and subjected to shaking fermentation for 30 h at 30°C under 250 rpm, and the titer of L-malate was measured by HPLC.

**Table 11 Flask shaking fermentation titer of malate of MA117-MA120 series strains**

| Strain | MA117-1 | MA117-2 | MA118-1 | MA118-2 |
|---|---|---|---|---|
| Malate, g/L | 0 | 0.96 | 1.04 | 1.28 |

| Strain | MA119-1 | MA119-2 | MA120-1 | MA120-2 |
|---|---|---|---|---|
| Malate, g/L | 0 | 1.12 | 1.19 | 1.42 |

## Claims

1. A genetically modified malate-producing yeast strain, having activity or enhanced activity of malate transport protein and having activity or enhanced activity of NADPH-dependent malate dehydrogenase (EC 1.1.1.82),
optionally further having activity or enhanced activity of at least one of: (i) pyruvate carboxylase (EC 6.4.1.1), (ii) phosphoenolpyruvate carboxykinase (EC 4.1.1.49), (iii) phosphoenolpyruvate carboxylase (EC 4.1.1.31) activity, preferably *Escherichia coli* phosphoenolpyruvate carboxylase, and (iv) biotin transport protein;
preferably, wherein said malate transport protein is selected from the group consisting of SpMAE1 protein, C4T318 protein and AsDct protein;
preferably, wherein said pyruvate carboxylase is derived from *Aspergillus oryzae* or *Pichia kudriαvzevii,*
preferably, wherein said NADPH-dependent malate dehydrogenase is derived from a plant, more preferably a C4 plant, more preferably a plant of the family Gramineae, Cyperaceae, Compositae, Euphorbiaceae, Chenopodiaceae, Portulacaceae or Amaranthaceae, or derived from the genus *Euglenα* or *Thermobacillus,* more preferably from *Sorghum bicolor, Zea mays, Saccharum officinarum, Pisum sativum, Cicer arietinum, Spinacia oleracea, Euglena gracilis* or *Methanothermobacter thermautotrophicus,*
more preferably, wherein said NADPH-dependent malate dehydrogenase is a *Sorghum bicolor-*derived NADPH-dependent malate dehydrogenase.

2. The genetically modified malate-producing yeast strain according to claim 1, further having reduced activity of or inactivated:
(i) pyruvate decarboxylase (EC 4.1.1.43), and/or
(ii) NAD-dependent glycerol-3-phosphate dehydrogenase (EC 1.1.1.8), and/or
(iii) orotidine 5'-phosphate decarboxylase (EC 4.1.1.23), and/or
(iv) monocarboxylate permease, and/or
(v) dicarboxylate transport protein, and/or
(vi) malic enzyme (EC 1.1.1.38), and/or
(vii) a bifunctional enzyme of oxaloacetate decarboxylation and 3-hydroxy-3-methylglutarate aldolase (EC 4.1.3.17 or 4.1.1.112).

3. The genetically modified malate-producing yeast strain according to claim 1 or 2, having at least one of the following:
(i) an over-expressed nucleic acid sequence encoding the NADPH-dependent malate dehydrogenase, preferably, said nucleic acid sequence encoding the NADPH-dependent malate dehydrogenase comprising the sequence of SEQ ID NO: 1 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having NADPH-dependent malate dehydrogenase activity,
(ii) an over-expressed nucleic acid sequence encoding the malate transport protein, preferably, said nucleic acid sequence encoding the malate transport protein comprising the sequence of SEQ ID NO: 2 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having malate transport protein activity,
(iii) an over-expressed nucleic acid sequence encoding the pyruvate carboxylase, preferably, said pyruvate carboxylase comprising the amino acid sequence of SEQ ID NO: 5 or an amino acid sequence having at least 75%, 80%, 85%, 90%, 91%, 92%, 93%, 94%, 95%, 96%, 97%, 98%, 99% or more identity thereto and having pyruvate carboxylase activity, or said nucleic acid sequence encoding the pyruvate carboxylase comprising the sequence of SEQ ID NO: 4 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having pyruvate carboxylase activity,
(iv) an over-expressed nucleic acid sequence encoding the phosphoenolpyruvate carboxylase, preferably, said nucleic acid sequence encoding the phosphoenolpyruvate carboxylase comprising the sequence of SEQ ID NO: 8 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having phosphoenolpyruvate carboxylase activity,
(v) an over-expressed nucleic acid sequence encoding the biotin transport protein, preferably, said nucleic acid sequence encoding the biotin transport protein comprising the sequence of SEQ ID NO: 3 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having biotin transport protein activity,
(vi) an over-expressed nucleic acid sequence encoding the phosphoenolpyruvate carboxykinase, preferably, said phosphoenolpyruvate carboxykinase comprises the amino acid sequence of SEQ ID NO: 6 or an amino acid sequence having at least 75%, 80%, 85%, 90%, 91%, 92%, 93%, 94%, 95%, 96%, 97%, 98%, 99% or more identity thereto and having phosphoenolpyruvate carboxykinase activity, or said nucleic acid sequence encoding the phosphoenolpyruvate carboxykinase comprising the sequence of SEQ ID NO: 7 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having phosphoenolpyruvate carboxykinase activity,
(vii) an endogenous gene encoding pyruvate decarboxylase being knocked out,
(viii) an endogenous gene encoding NAD-dependent glycerol-3-phosphate dehydrogenase being knocked out,
(ix) an endogenous gene encoding orotidine 5'-phosphate decarboxylase being knocked out,
(x) an endogenous gene encoding monocarboxylate permease being knocked out,
(xi) an endogenous gene encoding dicarboxylate transport protein being knocked out,
(xii) an endogenous gene encoding malic enzyme being knocked out,
(xiii) an endogenous gene encoding a bifunctional enzyme of oxaloacetate decarboxylation and 3-hydroxy-3-methylglutarate aldolase being knocked out,

4. The genetically modified malate-producing yeast strain according to any one of claims 1-3, wherein, in the genetically modified malate-producing yeast strain,
(a) a nucleic acid sequence encoding the NADPH-dependent malate dehydrogenase and a nucleic acid sequence encoding the malate transport protein are over-expressed; or
(b) a nucleic acid sequence encoding the NADPH-dependent malate dehydrogenase and a nucleic acid sequence encoding the malate transport protein are over-expressed, and an endogenous nucleic acid sequence encoding the pyruvate carboxylase and/or an endogenous nucleic acid sequence encoding NAD-dependent glycerol-3-phosphate dehydrogenase are knocked out; or
(c) a nucleic acid sequence encoding the NADPH-dependent malate dehydrogenase and a nucleic acid sequence encoding the malate transport protein are over-expressed, and an endogenous nucleic acid sequence encoding malic enzyme is knocked out; or
(d) the following nucleic acid sequences are over-expressed:
a nucleic acid sequence encoding the NADPH-dependent malate dehydrogenase;
a nucleic acid sequence encoding the malate transport protein; and
at least one of the following nucleic acid sequences:
a nucleic acid sequence encoding the biotin transport protein,
a nucleic acid sequence encoding *Escherichia coli* phosphoenolpyruvate carboxykinase,
a nucleic acid sequence encoding the phosphoenolpyruvate carboxylase, preferably a nucleic acid sequence encoding *Escherichia coli* phosphoenolpyruvate carboxylase,
a nucleic acid sequence encoding *Pichia kudriavzevii* phosphoenolpyruvate carboxykinase and
a nucleic acid sequence encoding the pyruvate carboxylase,
preferably, said pyruvate carboxylase is derived from *Aspergillus oryzae* or *Pichia kudriαvzevii,* more preferably, said nucleic acid sequence encoding the pyruvate carboxylase
encodes an amino acid sequence comprising the sequence of SEQ ID NO: 5, or encodes an amino acid sequence which has at least 75%, 80%, 85%, 90%, 91%, 92%, 93%, 94%, 95%, 96%, 97%, 98%, 99% or more identity to the sequence of SEQ ID NO: 5 and has the pyruvate carboxylase activity, or
comprises the sequence of SEQ ID NO: 4 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having pyruvate carboxylase activity;
(e) the following nucleic acid sequences are over-expressed:
a nucleic acid sequence encoding the NADPH-dependent malate dehydrogenase;
a nucleic acid sequence encoding the malate transport protein;
a nucleic acid sequence encoding *Escherichia coli* phosphoenolpyruvate carboxylase;
a nucleic acid sequence encoding the biotin transport protein; and
a nucleic acid sequence encoding *Aspergillus oryzae* pyruvate carboxylase,
and
an endogenous gene encoding pyruvate decarboxylase and/or an endogenous gene encoding NAD-dependent glycerol-3-phosphate dehydrogenase are knocked out,
preferably, the gene encoding *Aspergillus oryzae* pyruvate carboxylase comprising the sequence of SEQ ID NO: 4 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having pyruvate carboxylase activity.

5. The genetically modified malate-producing yeast strain according to any one of claims 1-4, wherein said malate-producing yeast strain is selected from the group consisting of the genera *Pichia, Rhodotroula, Saccharomyces, Yarrowia, Zygosaccharomyces, Torulopsis,* and *Candida,* preferably is selected from the group consisting of the genus *Pichia,* more preferably is *Pichia kudriαvzevii,* for example the *Pichia kudriavzevii* deposited in China General Microbiological Culture Collection Center (CGMCC) under the deposit number of CGMCC No. 20885.

6. A method for preparing a genetically modified malate-producing yeast strain, comprising conferring on the strain the activities of or enhancing in the strain the activities of malate transport protein and NADPH-dependent malate dehydrogenase (EC 1.1.1.82), optionally further comprising conferring or enhancing the activity of at least one of: (i) pyruvate carboxylase (EC 6.4.1.1) activity, (ii) phosphoenolpyruvate carboxykinase (EC 4.1.1.49) activity, (iii) phosphoenolpyruvate carboxylase (EC 4.1.1.31) activity, preferably *Escherichia coli* phosphoenolpyruvate carboxylase activity, and (iv) biotin transport protein activity,
preferably, said malate transport protein is selected from the group consisting of SpMAE1 protein, C4T318 protein and AsDct protein;
preferably, said pyruvate carboxylase is derived from *Aspergillus oryzae* or *Pichia kudriαvzevii,*
preferably, said NADPH-dependent malate dehydrogenase is derived from a plant, more preferably a C4 plant, more preferably a plant of the family Gramineae, Cyperaceae, Compositae, Euphorbiaceae, Chenopodiaceae, Portulacaceae or Amaranthaceae, or derived from the genus *Euglena* or *Thermobacillus,* more preferably from *Sorghum bicolor, Zea mays, Saccharum officinarum, Pisum sativum, Cicer arietinum, Spinacia oleracea, Euglena gracilis* or *Methanothermobacter thermautotrophicus,* more preferably, said NADPH-dependent malate dehydrogenase is a *Sorghum bicolor-derived* NADPH-dependent malate dehydrogenase.

7. The method according to claim 6, further comprising attenuating or inactivating in the strain:
(i) pyruvate decarboxylase (EC 4.1.1.43), and/or
(ii) NAD-dependent glycerol-3-phosphate dehydrogenase (EC 1.1.1.8), and/or
(iii) orotidine 5'-phosphate decarboxylase (EC 4.1.1.23), and/or
(iv) monocarboxylate permease; and/or
(v) dicarboxylate transport protein, and/or
(vi) malic enzyme (EC 1.1.1.38), and/or
(vii) a bifunctional enzyme of oxaloacetate decarboxylase and 3-hydroxy-3-methylglutarate aldolase (EC 4.1.3.17 or 4.1.1.112).

8. The method according to claim 6 or 7, comprising in the malate-producing yeast strain:
(i) over-expressing a nucleic acid sequence encoding the NADPH-dependent malate dehydrogenase, preferably, said nucleic acid sequence encoding the NADPH-dependent malate dehydrogenase comprises the sequence of SEQ ID NO: 1 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having NADPH-dependent malate dehydrogenase activity, and/or
(ii) over-expressing a nucleic acid sequence encoding the malate transport protein, preferably, said nucleic acid sequence encoding the malate transport protein comprises the sequence of SEQ ID NO: 2 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having malate transport protein activity, and/or
(ii) over-expressing a nucleic acid sequence encoding the pyruvate carboxylase, preferably, said nucleic acid sequence encoding the pyruvate carboxylase encodes the amino aicd sequence of SEQ ID NO: 5 or an amino acid sequence having at least 75%, 80%, 85%, 90%, 91%, 92%, 93%, 94%, 95%, 96%, 97%, 98%, 99% or more identity thereto and having the pyruvate carboxylase activity, or said nucleic acid sequence encoding the pyruvate carboxylase comprises the sequence of SEQ ID NO:4 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having pyruvate carboxylase activity, and/or
(iv) over-expressing a nucleic acid sequence encoding the phosphoenolpyruvate carboxylase, preferably, said nucleic acid sequence encoding the phosphoenolpyruvate carboxylase comprises the sequence of SEQ ID NO:8 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having phosphoenolpyruvate carboxylase activity, and/or
(v) over-expressing a nucleic acid sequence encoding the biotin transport protein, preferably, said nucleic acid sequence encoding the biotin transport protein comprises the sequence of SEQ ID NO: 3 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having biotin transport protein activity, and/or
(vi) over-expressing a nucleic acid sequence encoding the phosphoenolpyruvate carboxykinase, preferably, said nucleic acid sequence encoding the phosphoenolpyruvate carboxykinase comprises the sequence of SEQ ID NO: 7 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having phosphoenolpyruvate carboxykinase activity, or said phosphoenolpyruvate carboxykinase comprises the amino acid sequence of SEQ ID NO:6 or an amino acid sequence having at least 75%, 80%, 85%, 90%, 91%, 92%, 93%, 94%, 95%, 96%, 97%, 98%, 99% or more identity thereto and having phosphoenolpyruvate carboxykinase activity, and/or
(vii) knocking out an endogenous gene encoding pyruvate decarboxylase, and/or
(viii) knocking out an endogenous gene encoding NAD-dependent glycerol-3-phosphate dehydrogenase, and/or
(ix) knocking out an endogenous gene encoding orotidine 5'-phosphate decarboxylase, and/or
(x) knocking out an endogenous gene encoding monocarboxylate permease, and/or
(xi) knocking out an endogenous gene encoding dicarboxylate transport protein, and/or
(xii) knocking out an endogenous gene encoding malic enzyme, and/or
(xiii) knocking out an endogenous gene encoding a bifunctional enzyme of oxaloacetate decarboxylase and 3-hydroxy-3-methylglutarate aldolase.

9. The method according to any one of claims 6-8, comprising in the malate-producing yeast strain:
(a) over-expressing a nucleic acid sequence encoding the NADPH-dependent malate dehydrogenase and a nucleic acid sequence encoding the malate transport protein; or
(a) over-expressing a nucleic acid sequence encoding the NADPH-dependent malate dehydrogenase and a nucleic acid sequence encoding the malate transport protein, and knocking out an endogenous gene encoding pyruvate decarboxylase and an endogenous gene encoding NAD-dependent glycerol-3-phosphate dehydrogenase; or
(c) over-expressing a nucleic acid sequence encoding the NADPH-dependent malate dehydrogenase and a nucleic acid sequence encoding the malate transport protein, and knocking out an endogenous gene encoding malic enzyme;
(d) over-expressing the following nucleic acid sequences:
a nucleic acid sequence encoding the NADPH-dependent malate dehydrogenase;
a nucleic acid sequence encoding the malate transport protein;
at least one of the following nucleic acid sequences:
a nucleic acid sequence encoding the biotin transport protein,
a nucleic acid sequence encoding *Escherichia coli* phosphoenolpyruvate carboxykinase,
a nucleic acid sequence encoding the phosphoenolpyruvate carboxylase, preferably a nucleic acid sequence encoding *Escherichia coli* phosphoenolpyruvate carboxylase,
a nucleic acid sequence encoding *Pichia kudriavzevii* phosphoenolpyruvate carboxykinase and
a nucleic acid sequence encoding the pyruvate carboxylase, and
preferably, said pyruvate carboxylase is derived from *Aspergillus* oryzae or *Pichia kudriαvzevii,* more preferably, said nucleic acid sequence encoding the pyruvate carboxylase
encodes an amino acid sequence comprising the sequence of SEQ ID NO: 5, or encodes an amino acid sequence which has at least 75%, 80%, 85%, 90%, 91%, 92%, 93%, 94%, 95%, 96%, 97%, 98%, 99% or more identity to the sequence of SEQ ID NO: 5 and has pyruvate carboxylase activity, or
comprises the sequence of SEQ ID NO: 4 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having pyruvate carboxylase activity; or
(e) over-expressing the following nucleic acid sequences:
a nucleic acid sequence encoding the NADPH-dependent malate dehydrogenase;
a nucleic acid sequence encoding the malate transport protein;
at least one of the following nucleic acid sequences:
a nucleic acid sequence encoding *Escherichia coli* phosphoenolpyruvate carboxykinase,
a nucleic acid sequence encoding the phosphoenolpyruvate carboxylase,
a nucleic acid sequence encoding *Pichia kudriavzevii* phosphoenolpyruvate carboxykinase, preferably a nucleic acid sequence encoding *Escherichia coli* phosphoenolpyruvate carboxylase; and
a nucleic acid sequence encoding the pyruvate carboxylase,
and
knocking out an endogenous gene encoding pyruvate decarboxylase and an endogenous gene encoding NAD-dependent glycerol-3-phosphate dehydrogenase,
preferably, said pyruvate carboxylase is derived from *Aspergillus* oryzae or *Pichia kudriαvzevii,* more preferably, said nucleic acid sequence encoding the pyruvate carboxylase
encodes an amino acid sequence comprising the sequence of SEQ ID NO: 5, or encodes an amino acid sequence which has at least 75%, 80%, 85%, 90%, 91%, 92%, 93%, 94%, 95%, 96%, 97%, 98%, 99% or more identity to the sequence of SEQ ID NO: 5 and has pyruvate carboxylase activity, or
comprises the sequence of SEQ ID NO: 4 or a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having pyruvate carboxylase activity; or
(f) over-expressing the following nucleic acid sequences:
a nucleic acid sequence encoding the NADPH-dependent malate dehydrogenase;
a nucleic acid sequence encoding the malate transport protein;
a nucleic acid sequence encoding *Escherichia coli* phosphoenolpyruvate carboxylase;
a nucleic acid sequence encoding the biotin transport protein; and
a nucleic acid sequence encoding *Aspergillus oryzae* pyruvate carboxylase,
and
knocking out an endogenous gene encoding pyruvate decarboxylase and/or an endogenous gene encoding NAD-dependent glycerol-3-phosphate dehydrogenase,
preferably, said nucleic acid sequence encoding *Aspergillus oryzae* pyruvate carboxylase comprises the sequence of SEQ ID NO: 4 and a degenerate sequence thereof, or a nucleotide sequence having at least 75%, 80%, 85%, 90%, 91%, 92%, 93%, 94%, 95%, 96%, 97%, 98%, 99% or more identity thereto and encoding an amino acid sequence having pyruvate carboxylase activity.

10. The method according to any one of claims 6-9, wherein the malate-producing yeast strain is selected from the group consisting of the genera *Pichia, Rhodotroula, Saccharomyces, Yarrowia, Zygosaccharomyces, Torulopsis* and *Candida,* preferably is selected from the group consisting of the genus *Pichia,* more preferably is *Pichia kudriαvzevii,* for example the *Pichia kudriavzevii* deposited in China General Microbiological Culture Collection Center (CGMCC) under the deposit number of CGMCC No. 20885.

11. A method for producing L-malate, comprising culturing the genetically modified malate-producing yeast strain according to any one of claims 1-5 or a genetically modified malate-producing yeast strain prepared by the method according to any one of claims 6-10, preferably culturing at a pH value in the range of 2.0-3.5 and/or with no or less addition of a neutralizing agent, and optionally isolating and purifying the produced L-malate.

12. Use of the genetically modified yeast strain according to any one of claims 1-5 or a genetically modified yeast strain prepared by the method according to any one of claims 6-10 in the producuction of L-malate, preferably in the production of L-malate at a pH value in the range of 2.0-3.5 and/or with no or less addition of a neutralizing agent.
